# EUROPEAN PATENT SPECIFICATION

(11) **EP 2 809 338 B1**
(45) Date of publication and mention of the grant of the patent: **12.02.2020**
(21) Application number: 12842939.6
(22) Date of filing: 26.10.2012
(51) Int. Cl.: A61K 38/17, A61K 38/16, A61K 48/00, A61P 35/00

(54) **FLT3 MUTATIONS ASSOCIATED WITH DRUG RESISTANCE IN AML PATIENTS HAVING ACTIVATING MUTATIONS IN FLT3**
FLT3-MUTATIONEN IN VERBINDUNG MIT MEDIKAMENTENRESISTENZ BEI AML-PATIENTEN MIT AKTIVIERENDEN FLT3-MUTATIONEN
MUTATIONS DE FLT3 ASSOCIÉES À UNE PHARMACORÉSISTANCE CHEZ DES PATIENTS ATTEINTS DE LAM PRÉSENTANT DES MUTATIONS ACTIVATRICES DE FLT3

(30) Priority: 28.10.2011 US 201161553090 P
(43) Date of publication of application: 10.12.2014
(73) Proprietor: The Regents of the University of California, Oakland, CA 94607 (US)
(72) Inventor: SHAH, Neil Pravin, Mill Valley, California 94941 (US); SMITH, Catherine Choy, Menlo Park, California 94025 (US)
(74) Representative: J A Kemp LLP
(86) International application number: PCT/US2012/062241
(87) International publication number: WO 2013/063482

(56) References cited:
- Catherine C. Smith: "Abstract 4737: Saturation mutagenesis of FLT3-ITD: AC220-resistance-conferring kinase domain mutations are restricted to a limited number of residues and are cross-resistant to sorafenib in vitro", , 6 April 2011 (2011-04-06), XP055193890, Retrieved from the Internet: URL:http://cancerres.aacrjournals.org/cont ent/71/8_Supplement/4737 [retrieved on 2015-06-05]
- Catherinec Smith ET AL: "Saturation Mutagenesis of FLT3-ITD: AC220- Resistance-Conferring KinaseDomain Mutations are Restricted to a Limited Number of Residues and are Cross-Resistant to Sorafenibin vitro", , 22 August 2011 (2011-08-22), 22 August 2011 (2011-08-22), XP055193847, Retrieved from the Internet: URL:http://www.ambitbio.com/pdf/AACRSmithP resentationFinal.pdf [retrieved on 2015-06-05]
- CATHERINE C SMITH ET AL: "764: PLX3397 Is An Investigational Selective FLT3 Inhibitor That Retains Activity Against the Clinically-Relevant FLT3-ITD/F691L "Gatekeeper" Mutation in Vitro", BLOOD, vol. 118, no. 21, 18 November 2011 (2011-11-18), - 13 December 2011 (2011-12-13), page 347, XP055193708, US ISSN: 0006-4971
- Catherine C Smith et al.: "Paper: Analysis of in Vitro Activity of the Clinically-Active ABL/FLT3 Inhibitor Ponatinib (AP24534) Against AC220-Resistant FLT3-ITD Mutants", , 13 December 2011 (2011-12-13), XP055193831, Retrieved from the Internet: URL:https://ash.confex.com/ash/2011/webpro gram/Paper42526.html [retrieved on 2015-06-05]
- Els Lierman ET AL: "The ability of sorafenib to inhibit oncogenic PDGFR[beta] [beta] and FLT3 mutants and overcome resistance to other small molecule inhibitors", Accepted November Department of Molecular and Developmental Genetics Campus Gasthuisberg O&N1 Herestraat, 1 January 2007 (2007-01-01), XP055193881, Retrieved from the Internet: URL:http://www.haematologica.org/content/h aematol/92/1/27.full.pdf [retrieved on 2015-06-05]
- FLORIAN HEIDEL ET AL: "Bis(1 H -indol-2-yl)methanones are effective inhibitors of FLT3-ITD tyrosine kinase and partially overcome resistance to PKC412A in vitro", BRITISH JOURNAL OF HAEMATOLOGY, vol. 144, no. 6, 1 March 2009 (2009-03-01) , pages 865-874, XP055193867, ISSN: 0007-1048, DOI: 10.1111/j.1365-2141.2008.07567.x
- CHAO Q ET AL: "Identification of N-(5-tert-Butyl-isoxazol-3-yl-N'-{4-[7-(2- morpholin-4-yl-ethoxy)imidazo-[2,1-b][1,3] benzothiazol-2-yl]phenyl}urea Dihydrochloride (AC220), a unique potent, selective, and efficacious FMS-like tyrosine kinase-3 (FLT3) inhibitor", JOURNAL OF MEDICINAL CHEMISTRY, AMERICAN CHEMICAL SOCIETY, US, vol. 52, 10 December 2009 (2009-12-10), pages 7808-7816, XP002615038, ISSN: 0022-2623, DOI: 10.1021/JM9007533 [retrieved on 2009-09-16]
- Mohammad Azam ET AL: "Mechanisms of Autoinhibition and STI-571/Imatinib Resistance Revealed by Mutagenesis of BCR-ABL", Cell, 1 January 2003 (2003-01-01), pages 831-843, XP055547946, United States DOI: 10.1016/S0092-8674(03)00190-9 Retrieved from the Internet: URL:https://www.sciencedirect.com/science/ article/pii/S0092867403001909/pdfft?md5=2d 29ec8643f576f14516d9a6012050c4&pid=1-s2.0- S0092867403001909-main.pdf

## Description

### BACKGROUND OF THE INVENTION

Efforts to develop highly effective targeted cancer therapeutics involve distinguishing disease-associated "driver" mutations, which play critical causative roles in malignancy pathogenesis, from "passenger" mutations that are dispensable for cancer initiation and/or maintenance. Translational studies of clinically active targeted therapeutics can distinguish "driver" from "passenger" lesions and provide valuable insights into human disease biology. Activating in tandem duplication (ITD) mutations in FLT3 (FLT3-ITD) are detected in approximately 30% of acute myeloid leukemia (AML) patients and are associated with a poor prognosis¹. Abundant scientific^{2,3} and clinical^{1,4,5} evidence suggests that FLT3-ITD mutations likely represent "passenger" lesions.

C. C. Smith described saturation mutagenesis of FLT3-ITD and described that AC220-resistance-conferring kinase domain mutations are restricted to a limited number of residues and are cross-resistant to sorafenib *in vitro* (Abstract 4737, 6 April 2011, URL:http://cancerres.aacrjournals.org/content/71/8_Supplement/4737; 22 August 2011, URL:http://www.ambitbio.com/pdf/AACRSmithPresentationFinal.pdf). C. C. Smith et al (2011, Blood, 118(21):347) described that PLX3397 is an investigational selective FLT3 inhibitor that retains activity against the clinically-relevant FLT3-ITD/F691L "gatekeeper" mutation *in vitro.* C. C. Smith *et al.* described an analysis of in vitro activity of the clinically-active ABL/FLT3 inhibitor ponatinib (AP24534) against AC220-resistant FLT3-ITD mutants (13 December 2011, URL:https://ash.confex.com/ash/2011/webprogram/Paper42526.html).

In previous clinical studies, numerous investigational FLT3 TKIs have failed to achieve complete remissions when employed as monotherapy (0 of 134 complete remissions in AML patients in Phase II studies, collectively)⁶⁻⁸, although the extent to which these agents achieved biochemically potent and/or sustained FLT3 inhibition *in vivo* is not known. Perhaps the most compelling data to suggest that activated FLT3 could represent a "driver" mutation in AML was the identification of a FLT3 kinase domain mutation conferring moderate resistance to the multikinase inhibitor PKC412 in a single patient who relapsed after achieving ≥ 50% reduction in peripheral blood and/or bone marrow blasts on PKC412 treatment, although five additional patients evaluated in that study did not have resistant mutations⁹. While the broad-spectrum multikinase inhibitor sorafenib has recently been reported to achieve remissions in FLT3-ITD+ AML patients in a small compassionate use study¹⁰, it is unclear whether its mechanism of action involves inhibition of FLT3 or a distinct kinase. Indeed, two patients who relapsed on sorafenib after initially responding had no detectable FLT3 kinase domain mutations¹¹.

AC220 (quizartinib) is a clinically active investigational inhibitor with selectivity towards FLT3, KIT, PDGFR and RET¹². A multinational phase II monotherapy of AC220 study is currently ongoing. A recent interim analysis of 53 patients evaluable for efficacy documented a composite complete remission (<5 percent bone marrow blasts) rate of 45 percent in relapsed/refractory FLT3-ITD+ AML patients¹³.

Multiple factors have been implicated as possible underlying causes of resistance to FLT3 inhibitors. Indeed, unlike the case for ABL inhibitors, studies of resistance profiles of three FLT3 inhibitors, PKC412, SU5614, and sorafenib showed nonoverlapping mechanisms of resistance for the three inhibitors (von Bubnoff et al, Cancer Res. 69:3032-3041, 1009).

There is thus a need to understand mechanisms that underlie resistance that can emerge following treatment of an AML patient with the FLT3 inhibitor AC220.

### BRIEF SUMMARY OF THE INVENTION

This invention is based, in part, upon the identification of mutations at residues within the FLT3-ITD kinase domain that confer resistance to the chemotherapeutic drug AC220 (quizartinib), the first investigational FLT3 inhibitor to demonstrate convincing clinical activity in FLT3-ITD⁺ AML. These findings demonstrate that FLT3-ITD is a "driver" lesion in a substantial proportion of AML patients, and therefore represents a valid therapeutic target in human AML. Further, clinically relevant AC220-resistant FLT3-ITD kinase domain mutants represent high-value therapeutic targets for future FLT3 inhibitor development efforts.

In one aspect, the invention thus provides a method of identifying an AML patient undergoing treatment with AC220 that has an increased likelihood of relapse, wherein the patient has an initial activating mutation in a FLT3 gene, the method comprising detecting the presence of at least a second mutation (*i.e.,* a resistance mutation) in the FLT3 gene in an AML cell sample from the patient, wherein the second mutation results in an amino acid substitution at position F691, D835, or Y842 of FLT3. In some embodiments, the second mutation activates FLT3. In some embodiments, the initial activating mutation is an in tandem duplication (ITD) mutation. In some embodiments, the initial activating mutation is a substitution at Y842. In some embodiments, *e.g.,* where the initial activating mutation is at Y842, the resistance mutation is a substitution at F691 or D835. In some embodiments, the method comprises determining the presence of the second mutation in a FLT3 gene at a codon that encodes F691, D835, or Y842. In some embodiments, a method of the invention comprises sequencing a nucleic acid amplified from the region of the FLT3 gene that comprises the codon. In some embodiments, the second mutation is at D835. In some embodiments, the second mutation is D835Y, D835V, or D835F. In some embodiments, the second mutation is at F691. In some embodiments, the second mutation is F691L. In some embodiments, the mutation is F691I. In some embodiments, the patient has an amino acid substitution at position F691 and an amino acid substitution at position D835. In some embodiments, the second mutation is at position Y842. In some embodiments, the mutation is Y842C or Y842H. In some aspects, the second mutation is a mutation at position A848, N841, or D839. In some aspects, the second mutation sis A848P, N841K, or D839V. In some embodiments, the AML cell sample is obtained from blood. In some embodiments, the AML cell sample is obtained from bone marrow. In some embodiments, the AML cell sample is obtained from a metastatic site, *e.g.,* from the central nervous system, *e.g.,* the spinal cord or brain.

In some embodiments, the mutation is detected using single molecule sequencing (e.g., the True Single Molecule Sequencing (tSMS™) sequencing platform (Helicos BioSciences Corporation); or Real Time Single Molecule Sequencing (SMRT™) sequencing platform (Pacific Biosciences Incorporated).

In some aspects, a method of the disclosure comprises identifying an AC220 resistance mutation in an AML cell sample from a patient, *e.g.,* an amino acid substitution at position F691, D835, or Y842, e.g., D835Y, D835V, D835F, or F691L, and administering a therapeutic agent other than AC220 to the patient. In some aspects, the alternative therapeutic agent is a drug that is active against the resistance mutation. In some aspects, the therapeutic agent is ponatinib (Ariad Pharmaceuticals), PLX3397 (Plexxikon, Inc), G749 (Genosco, Cambridge, MA), or crenolanib (AROG Pharmaceuticals). In some aspects, the patient has a resistance mutation at position A848, N841, or D839. In some aspects, the resistance mutation is A848P, N841K, F691I, Y842H, Y842C, or D839V.

In a further aspect, the disclosure also provides a method of monitoring progression of AML in a patient that has an initial activating mutation in a FLT3 gene and is subjected to AC220 therapy, the method comprising detecting a change in the number of cells that comprise a second mutation in FLT3, wherein the second mutation is at a codon that encodes F691, D835, or Y842, where the change in the number of cells having the second mutation is indicative of the patient's response to the AC220 therapy. In some aspects, the mutation is D835Y, D835V, D835F, or F691L. In some aspects, the mutation is A848P, N841K, F691I, Y842H, Y842C, or D839V.

In another aspect, the disclosure provides methods of identifying molecules that inhibit the mutant FLT3 protein. In some aspects, such a method comprises a step of identifying a compound that specifically binds to the mutant FLT3 protein that has a resistance mutation as described herein above.

In another aspect, the disclosure provides a method of inhibiting growth and/or proliferation of AML cells, the method comprising administering a further therapeutic agent that inhibits FLT3 tyrosine kinase to an AC220-treated patient that has an initial activating mutation in a FLT3 gene, *e.g.,* an ITD mutation, and is determined to have a second mutation at a codon that encodes F691, D835, or Y842. In some aspects, the mutation is D835Y, D835V, D835F, or F691L. In some aspects, the mutation is at position A848, N841, or D839. In some aspects, the mutation is A848P, N841K, F691I, Y842H, Y842C, or D839V. In some aspects, the inhibitor is ponatinib (Ariad Pharmaceuticals), PLX3397 (Plexxikon, Inc), G749 (Genosco, Cambridge, MA), or crenolanib (AROG Pharmaceuticals). In some aspects, a patient treated with PLX3397 has a resistance mutation F691L.

### BRIEF DESCRIPTION OF THE DRAWINGS

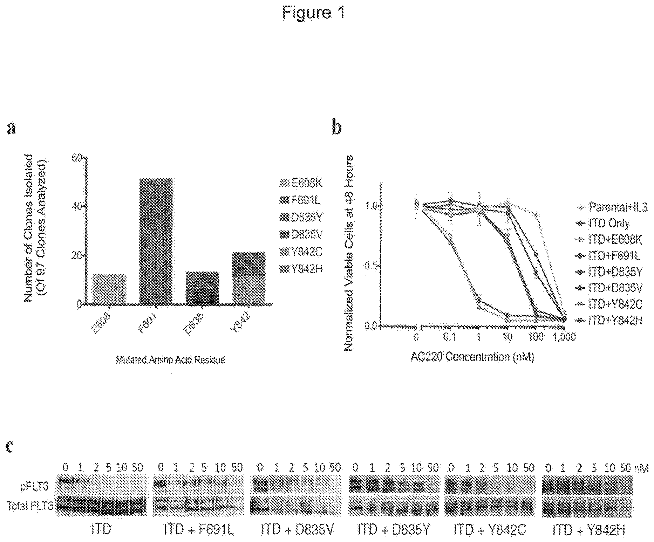

Figure 1. Mutation Screen of FLT3-ITD Reveals Secondary Kinase Domain Mutations that Cause Varying Degrees of Resistance to AC220. (A) Numbers of independent AC220-resistant Ba/F3/FLT3-ITD subpopulations with amino acid substitution at the indicated residue obtained from a saturation mutagenesis assay (n=97 clones analyzed). (B) Normalized cell viability of Ba/F3 populations stably expressing FLT3-ITD mutant isoforms after 48 hours in various concentrations of AC220. (C) Western blot analysis using anti-phospho-FLT3 or anti-FLT3 antibody performed on lysates prepared from IL-3-independent Ba/F3 populations infected with retroviruses expressing the FLT3 mutant isoforms indicated. Cells were exposed to the concentrations of AC220 indicated for 90 minutes.

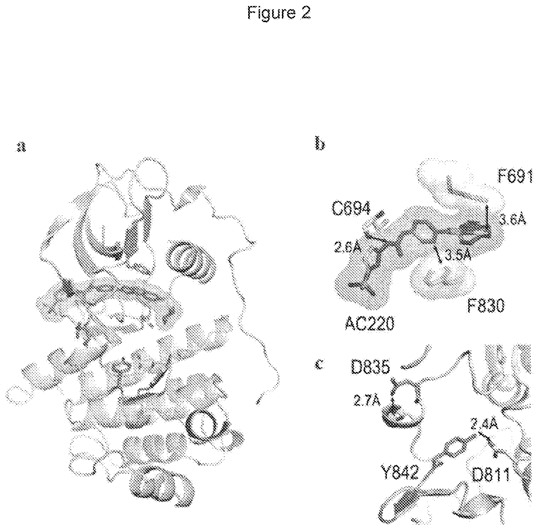

Figure 2: Modeling of FLT3-AC220 interactions. (A) The computational docking model of the AC220 bound FLT3 kinase domain. AC220 (blue) is presented in both stick mode and surface mode. The protein is shown in cartoon presentation. Amino acid residues that confer AC220 resistance when mutated (F691, D835, Y842) are depicted in orange sticks and the DFG motif is shown in white sticks. The model was generated using AutoDock [37see materials and methods] and the illustration was made in PyMol (Delano Scientific). (B) Surface and stick presentation of AC220 and the AC220-interacting interacting residues on FLT3. The carbonyl oxygen of C694 forms a hydrogen bond with an AC220 amide group. F691, F830 and AC220 form tight *π*-*π* stacking interactions. (C) The structure of the folded activation loop. Residues D835 and Y842 are depicted in orange sticks and their interacting residues on FLT3 are shown in white sticks.

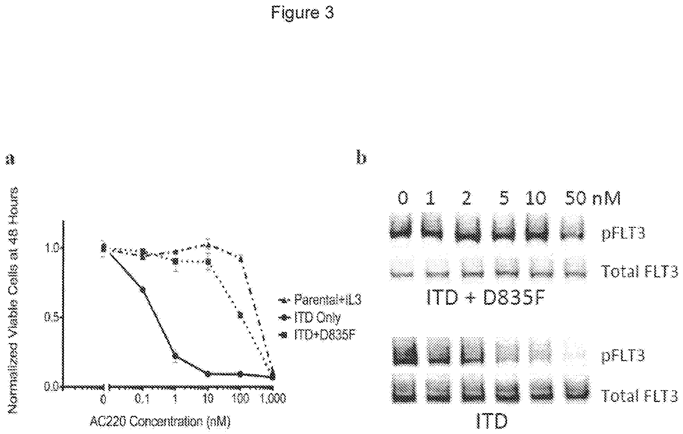

Figure 3: D835F Mutation Confers Resistance to ACC220 in vitro. (A) Normalized cell viability of Ba/F3 populations stably expressing FLT3-ITD or FLT3-ITD/D835F after 48 hours in various concentrations of AC220. (B) Western blot analysis using an anti-phospho-FLT3 and anti-FLT3 antibodies performed on lysates prepared from IL-3-independent Ba/F3 populations infected with retroviruses expressing FLT3-ITD and FLT3-ITD/D835F. Cells were exposed to the concentrations of AC220 indicated for 90 minutes.

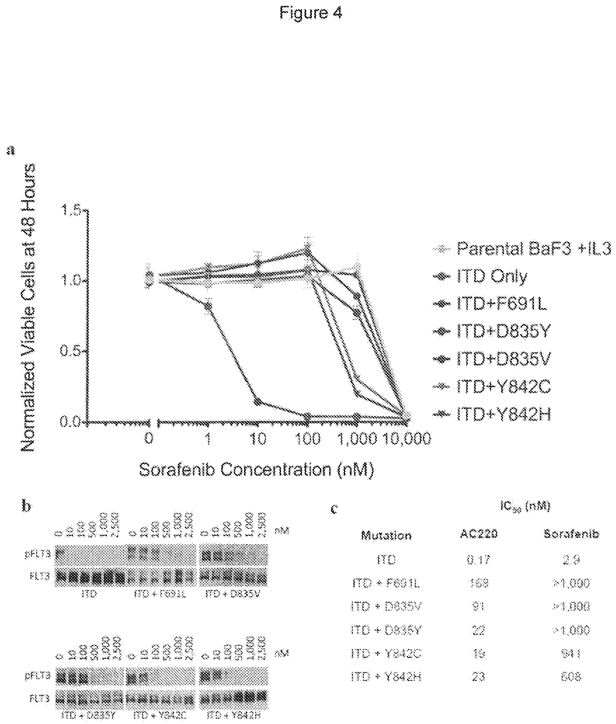

Figure 4: AC220-resistant FLT3-ITD Mutant Isoforms Confer Cross-Resistance to Sorafenib in vitro. (A) Normalized cell viability of Ba/F3 populations stably expressing AC220-resistant FLT3-ITD mutant isoforms after 48 hours in various concentrations of sorafenib. (B) Western blot analysis using anti-phospho-FLT3 or anti-FLT3 antibody performed on lysates prepared from IL-3-independent Ba/F3 populations infected with retroviruses expressing the FLT3 mutant isoforms indicated. Cells were exposed to the concentrations of sorafenib indicated for 90 minutes. (C) Calculated IC50 values for proliferation of Ba/F3 cells expressing FLT3 mutant isoforms grown in the presence of AC220 and sorafenib.

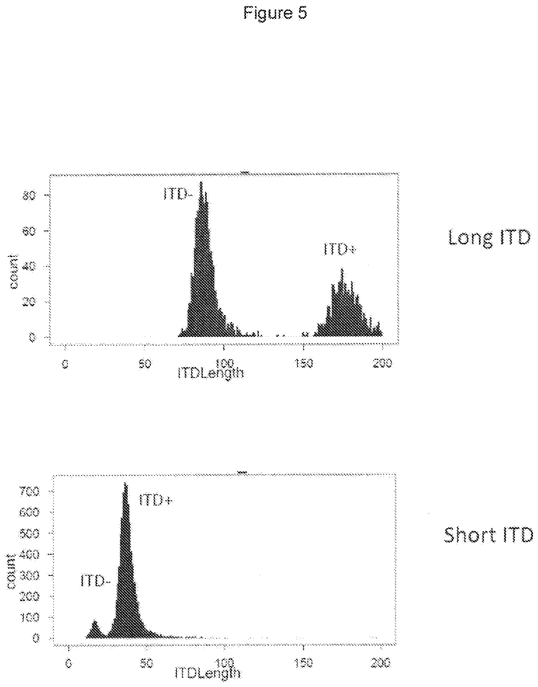

Figure 5: Example of Length Distribution of ITD Regions in a Patient Sample. Two distinct peaks identify ITD-/ITD+ subreads unambiguously.

### DETAILED DESCRIPTION OF THE INVENTION

### Definitions

The term "FLT3" refers to a receptor tyrosine kinase that plays a role in regulating hematopoiesis. "FLT3" is also known as CD135, stem cell tyrosine kinase 1 (STK1), or fetal liver kinase 2 (FLK2). FLT3 is a member of the type III receptor tyrosine kinase family that includes KIT, FMS, and platelet-derived growth factor receptor (PDGFR). The receptor has an extracellular domain that includes five immunoglobulin-like domains, a transmembrane domain and an intracellular domain that includes a kinase domain. A FLT3 receptor is activated by binding of the FMS-related tyrosine kinase 3 ligand to the extracellular domain, which induces homodimer formation in the plasma membrane leading to autophosphorylation of the receptor. The activated receptor kinase subsequently phosphorylates and activates multiple cytoplasmic effector molecules in pathways involved in apoptosis, proliferation, and differentiation of hematopoietic cells in bone marrow. Mutations that result in the constitutive activation of this receptor result in leukemia, *e.g.,* acute myeloid leukemia and acute lymphoblastic leukemia. The term "FLT3" as used herein encompasses nucleic acid and polypeptide polymorphic variants, alleles, mutants, and fragments. FLT3 sequences are well known in the art. Human FLT3 protein sequence has the UniProtKB accession number P36888. An example of a human FLT3 polypeptide sequences is available under the reference sequences NP_004110.2 in the NCBI polypeptide sequence database. Example of a representative FLT3 polynucleotide sequence is available in the NCBI database under accession number NM_004119.2. The polynucleotide sequence shown under accession number NM_004119.2 is provided as SEQ ID NO:1 as an illustrative nucleotide sequence. An illustrative polypeptide sequence from accession number NP_004110.2 is shown in SEQ ID NO:2. As understood in the art, the term "FLT3" includes variants, such as polymorphic variants, encoded by a FLT3 gene localized to human Entrez Gene cytogenetic band 13q12 (Ensembl cytogenetic band: 13q12.2; HGNC cytogenetic band: 13q12) and corresponds to positions 28.58 Mb-28.67 Mb UCSC Genome Browser on Human Feb. 2009 (GRCh37/hg19) Assembly. For example, the SNP database shows that single nucleotide polymorphisms have been identified in *FLT3* genes.

A FLT3 "activating mutation" in the context of this invention refers to a mutation that leads to constitutive activity of the kinase domain. As used herein, a "resistance" mutation refers to a mutation that leads to drug resistance. In the current invention, the drug is AC220. In the context of this invention "detecting a resistance mutation at a codon that encodes F691, D835, or Y842"; or "detecting a resistance mutation at F691, D835, or Y842" means that an AML patient that is undergoing AC220 therapy and is being evaluated in accordance with the methods of the invention has an initial activating FLT3 mutation, typically an ITD mutation. The resistance mutation may also be regarded as a "second" mutation, relative to the "initial" mutation. In some embodiments, the "second" or "resistance" mutation detected in accordance with the invention is a mutation at F691, D835, or Y842. In some embodiments, the "second" or "resistance" mutation is D835Y, D835V, D835F, or F691L. In some aspects, the "second" or "resistance" mutation is at position A848, N841, or D839. In some aspects, the "second" or "resistance" mutation is A848P, N841K, F691I, Y842H, Y842C, or D839V. In some embodiments, the resistance mutation may also activate FLT3, *e.g.,* a mutation at D835 or Y842. In some embodiments, the patient may have more than one resistance mutations, *e.g.,* a mutation at D835 and a mutation at F691.

The term "acute myeloid leukemia" ("AML"), also known as "acute myelogenous leukemia", refers to a cancer of the myeloid line of blood cells, characterized by the rapid growth of abnormal white blood cells that accumulate in the bone marrow and interfere with the production of normal blood cells. AML may be classified using either the World Health Organization classification (Vardiman JW, Harris NL, Brunning RD (2002). "The World Health Organization (WHO) classification of the myeloid neoplasms". Blood 100 (7): 2292-302); or the FAB classification (Bennett J, Catovsky D, Daniel M, Flandrin G, Galton D, Gralnick H, Sultan C (1976). "Proposals for the classification of the acute leukaemias. French-American-British (FAB) co-operative group". Br J Haematol 33 (4): 451-8.) In the context of this invention, an "AML patient" refers to a human.

The terms "tumor" or "cancer" in an animal refers to the presence of cells possessing characteristics such as atypical growth or morphology, including uncontrolled proliferation, immortality, metastatic potential, rapid growth and proliferation rate, and certain characteristic morphological features. "Cancer" includes both benign and malignant neoplasms. The term "neoplastic" refers to both benign and malignant atypical growth.

"Biological sample" as used herein refers to a sample that comprises AML cells obtained from a patient that has AML. The sample may be a biopsy, which refers to any type of biopsy, such as needle biopsy, fine needle biopsy, surgical biopsy, etc, *e.g.,* from bone marrow. In some embodiments, the biological sample is obtained from blood.

"Providing a biological sample" means to obtain a biological sample for use in methods described in this invention. Most often, this will be done by removing a sample of AML cells from a patient, but can also be accomplished by using previously isolated cells (e.g., isolated by another person, at another time, and/or for another purpose).

The terms "isolated," "purified," or "biologically pure" refer to material that is substantially or essentially free from components that normally accompany it as found in its native state. Purity and homogeneity are typically determined using analytical chemistry techniques such as polyacrylamide gel electrophoresis or high performance liquid chromatography. A protein or nucleic acid that is the predominant species present in a preparation is substantially purified. In particular, an isolated nucleic acid is separated from some open reading frames that naturally flank the gene and encode proteins other than protein encoded by the gene. The term "purified" in some embodiments denotes that a nucleic acid or protein gives rise to essentially one band in an electrophoretic gel. Preferably, it means that the nucleic acid or protein is at least 85% pure, more preferably at least 95% pure, and most preferably at least 99% pure. "Purify" or "purification" in other embodiments means removing at least one contaminant from the composition to be purified. In this sense, purification does not require that the purified compound be homogenous, e.g., 100% pure.

The terms "polypeptide," "peptide" and "protein" are used interchangeably herein to refer to a polymer of amino acid residues. The terms apply to amino acid polymers in which one or more amino acid residue is an artificial chemical mimetic of a corresponding naturally occurring amino acid, as well as to naturally occurring amino acid polymers, those containing modified residues, and non-naturally occurring amino acid polymer.

The term "amino acid" refers to naturally occurring and synthetic amino acids, as well as amino acid analogs and amino acid mimetics that function similarly to the naturally occurring amino acids. Naturally occurring amino acids are those encoded by the genetic code, as well as those amino acids that are later modified, e.g., hydroxyproline, γ-carboxyglutamate, and O-phosphoserine. Amino acid analogs refers to compounds that have the same basic chemical structure as a naturally occurring amino acid, e.g., an α carbon that is bound to a hydrogen, a carboxyl group, an amino group, and an R group, e.g., homoserine, norleucine, methionine sulfoxide, methionine methyl sulfonium. Such analogs may have modified R groups (e.g., norleucine) or modified peptide backbones, but retain the same basic chemical structure as a naturally occurring amino acid. Amino acid mimetics refers to chemical compounds that have a structure that is different from the general chemical structure of an amino acid, but that functions similarly to a naturally occurring amino acid.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, conservatively modified variants refers to those nucleic acids which encode amino acid sequences that are identical or share similar chemical properties to the native amino acid, or where the nucleic acid does not encode an amino acid sequence, to essentially identical or associated, e.g., naturally contiguous, sequences. Because of the degeneracy of the genetic code, a large number of functionally identical nucleic acids encode most proteins. For instance, the codons GCA, GCC, GCG and GCU all encode the amino acid alanine. Thus, at every position where an alanine is specified by a codon, the codon can be altered to another of the corresponding codons described without altering the encoded polypeptide. Such nucleic acid variations are "silent variations," which are one species of conservatively modified variations. Every nucleic acid sequence herein which encodes a polypeptide also describes silent variations of the nucleic acid. One of skill will recognize that in certain contexts each codon in a nucleic acid (except AUG, which is ordinarily the only codon for methionine, and TGG, which is ordinarily the only codon for tryptophan) can be modified to yield a functionally identical molecule. Accordingly, often silent variations of a nucleic acid which encodes a polypeptide is implicit in a described sequence with respect to the expression product, but not with respect to actual probe sequences.

As to amino acid sequences, one of skill will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the substitution of an amino acid with a chemically similar amino acid. Conservative substitution tables providing functionally similar amino acids are well known in the art. Such conservatively modified variants are in addition to and do not exclude polymorphic variants, interspecies homologs, and alleles of the invention.typically conservative substitutions for one another: 1) Alanine (A), Glycine (G); 2) Aspartic acid (D), Glutamic acid (E); 3) Asparagine (N), Glutamine (Q); 4) Arginine (R), Lysine (K); 5) Isoleucine (I), Leucine (L), Methionine (M), Valine (V); 6) Phenylalanine (F), Tyrosine (Y), Tryptophan (W); 7) Serine (S), Threonine (T); and 8) Cysteine (C), Methionine (M) (*see, e.g.,* Creighton, Proteins (1984)).

"Nucleic acid" or "oligonucleotide" or "polynucleotide" or grammatical equivalents used herein means at least two nucleotides covalently linked together. Oligonucleotides are typically from about 5, 6, 7, 8, 9, 10, 12, 15, 25, 30, 40, 50 or more nucleotides in length, up to about 100 nucleotides in length. Nucleic acids and polynucleotides are a polymers of any length, including longer lengths, e.g., 200, 300, 500, 1000, 2000, 3000, 5000, 7000, 10,000, etc. A nucleic acid of the present invention will generally contain phosphodiester bonds, although in some cases, nucleic acid analogs are included that may have alternate backbones, comprising, *e.g.,* phosphoramidate, phosphorothioate, phosphorodithioate, or O-methylphophoroamidite linkages (see Eckstein, Oligonucleotides and Analogues: A Practical Approach, Oxford University Press); and peptide nucleic acid backbones and linkages. Other analog nucleic acids include those with positive backbones; non-ionic backbones, and non-ribose backbones, including those described in U.S. Patent Nos. 5,235,033 and 5,034,506, and Chapters 6 and 7, ASC Symposium Series 580, *Carbohydrate Modifications in Antisense Research,* Sanghui & Cook, eds.. Nucleic acids containing one or more carbocyclic sugars are also included within one definition of nucleic acids. Modifications of the ribose-phosphate backbone may be done for a variety of reasons, *e.g.,* to increase the stability and half-life of such molecules in physiological environments or as probes on a biochip. Mixtures of naturally occurring nucleic acids and analogs can be made; alternatively, mixtures of different nucleic acid analogs, and mixtures of naturally occurring nucleic acids and analogs may be made.

A variety of references disclose such nucleic acid analogs, including, for example, phosphoramidate (Beaucage et al., Tetrahedron 49(10):1925 (1993) and references therein; Letsinger, J. Org. Chem. 35:3800 (1970); Sprinzl et al., Eur. J. Biochem. 81:579 (1977); Letsinger et al., Nucl. Acids Res. 14:3487 (1986); Sawai et al, Chem. Lett. 805 (1984), Letsinger et al., J. Am. Chem. Soc. 110:4470 (1988); and Pauwels et al., Chemica Scripta 26:141 91986)), phosphorothioate (Mag et al., Nucleic Acids Res. 19:1437 (1991); and U.S. Patent No. 5,644,048), phosphorodithioate (Briu et al., J. Am. Chem. Soc. 111:2321 (1989), O-methylphophoroamidite linkages (see Eckstein, Oligonucleotides and Analogues: A Practical Approach, Oxford University Press), and peptide nucleic acid backbones and linkages (see Egholm, J. Am. Chem. Soc. 114:1895 (1992); Meier et al., Chem. Int. Ed. Engl. 31:1008 (1992); Nielsen, Nature, 365:566 (1993); Carlsson et al., Nature 380:207 (1996)). Other analog nucleic acids include those with positive backbones (Denpcy et al., Proc. Natl. Acad. Sci. USA 92:6097 (1995); non-ionic backbones (U.S. Patent Nos. 5,386,023, 5,637,684, 5,602,240, 5,216,141 and 4,469,863; Kiedrowshi et al., Angew. Chem. Intl. Ed. English 30:423 (1991); Letsinger et al., J. Am. Chem. Soc. 110:4470 (1988); Letsinger et al., Nucleoside & Nucleotide 13:1597 (1994); Chapters 2 and 3, ASC Symposium Series 580, "Carbohydrate Modifications in Antisense Research", Ed. Y.S. Sanghui and P. Dan Cook; Mesmaeker et al., Bioorganic & Medicinal Chem. Lett. 4:395 (1994); Jeffs et al., J. Biomolecular NMR 34:17 (1994); Tetrahedron Lett. 37:743 (1996)) and non-ribose backbones, including those described in U.S. Patent Nos. 5,235,033 and 5,034,506, and Chapters 6 and 7, ASC Symposium Series 580, "Carbohydrate Modifications in Antisense Research", Ed. Y.S. Sanghui and P. Dan Cook. Nucleic acids containing one or more carbocyclic sugars are also included within one definition of nucleic acids (see Jenkins et al., Chem. Soc. Rev. (1995) pp 169-176). Several nucleic acid analogs are described in Rawls, C & E News June 2, 1997 page 35.

Other analogs include peptide nucleic acids (PNA) which are peptide nucleic acid analogs. These backbones are substantially non-ionic under neutral conditions, in contrast to the highly charged phosphodiester backbone of naturally occurring nucleic acids. This results in two advantages. First, the PNA backbone exhibits improved hybridization kinetics. PNAs have larger changes in the melting temperature (Tₘ) for mismatched versus perfectly matched basepairs. DNA and RNA typically exhibit a 2-4°C drop in Tₘ for an internal mismatch. With the non-ionic PNA backbone, the drop is closer to 7-9°C. Similarly, due to their non-ionic nature, hybridization of the bases attached to these backbones is relatively insensitive to salt concentration. In addition, PNAs are not degraded by cellular enzymes, and thus can be more stable.

The nucleic acids may be single stranded or double stranded, as specified, or contain portions of both double stranded or single stranded sequence. As will be appreciated by those in the art, the depiction of a single strand also defines the sequence of the complementary strand; thus the sequences described herein also provide the complement of the sequence. Unless otherwise indicated, a particular nucleic acid sequence also implicitly encompasses conservatively modified variants thereof (*e.g.,* degenerate codon substitutions) and complementary sequences, as well as the sequence explicitly indicated. The nucleic acid may be DNA, both genomic and cDNA, RNA or a hybrid, where the nucleic acid may contain combinations of deoxyribo- and ribo-nucleotides, and combinations of bases, including uracil, adenine, thymine, cytosine, guanine, inosine, xanthine hypoxanthine, isocytosine, isoguanine, etc. "Transcript" typically refers to a naturally occurring RNA, e.g., a pre-mRNA, hnRNA, or mRNA. As used herein, the term "nucleoside" includes nucleotides and nucleoside and nucleotide analogs, and modified nucleosides such as amino modified nucleosides. In addition, "nucleoside" includes non-naturally occurring analog structures. Thus, e.g. the individual units of a peptide nucleic acid, each containing a base, are referred to herein as a nucleoside.

A "label" or a "detectable moiety" is a composition detectable by spectroscopic, photochemical, biochemical, immunochemical, chemical, or other physical means. For example, useful labels include ³²P, fluorescent dyes, electron-dense reagents, enzymes (*e.g.,* as commonly used in an ELISA), biotin, digoxigenin, or haptens and proteins or other entities which can be made detectable, *e.g.,* by incorporating a radiolabel into the peptide or used to detect antibodies specifically reactive with the peptide. The labels may be incorporated into the KIT nucleic acids, proteins and antibodies at any position. Any method known in the art for conjugating the antibody to the label may be employed, *e.g.,* using methods described in Hermanson, Bioconjugate Techniques 1996, Academic Press, Inc., San Diego.

A "labeled nucleic acid probe or oligonucleotide" is one that is bound, either covalently, through a linker or a chemical bond, or noncovalently, through ionic, van der Waals, electrostatic, or hydrogen bonds to a label such that the presence of the probe may be detected by detecting the presence of the label bound to the probe. Alternatively, method using high affinity interactions may achieve the same results where one of a pair of binding partners binds to the other, e.g., biotin, streptavidin.

As used herein a "nucleic acid probe or oligonucleotide" is defined as a nucleic acid capable of binding to a target nucleic acid of complementary sequence through one or more types of chemical bonds, usually through complementary base pairing, usually through hydrogen bond formation. As used herein, a probe may include natural (i.e., A, G, C, or T) or modified bases (7-deazaguanosine, inosine, etc.). In addition, the bases in a probe may be joined by a linkage other than a phosphodiester bond, so long as it does not functionally interfere with hybridization. Thus, *e.g.,* probes may be peptide nucleic acids in which the constituent bases are joined by peptide bonds rather than phosphodiester linkages. It will be understood by one of skill in the art that probes may bind target sequences lacking complete complementarity with the probe sequence depending upon the stringency of the hybridization conditions. The probes are preferably directly labeled as with isotopes, chromophores, lumiphores, chromogens, or indirectly labeled such as with biotin to which a streptavidin complex may later bind. By assaying for the presence or absence of the probe, one can detect the presence or absence of the select sequence or subsequence. Diagnosis or prognosis may be based at the genomic level, or at the level of RNA or protein expression.

The term "recombinant" when used with reference, e.g., to a cell, or nucleic acid, protein, or vector, indicates that the cell, nucleic acid, protein or vector, has been modified by the introduction of a heterologous nucleic acid or protein or the alteration of a native nucleic acid or protein, or that the cell is derived from a cell so modified. Thus, *e.g.,* recombinant cells express genes that are not found within the native (non-recombinant) form of the cell or express native genes that are otherwise abnormally expressed, under expressed or not expressed at all. By the term "recombinant nucleic acid" herein is meant nucleic acid, originally formed *in vitro,* in general, by the manipulation of nucleic acid, *e.g.,* using polymerases and endonucleases, in a form not normally found in nature. Similarly, a "recombinant protein" is a protein made using recombinant techniques, i.e., through the expression of a recombinant nucleic acid as depicted above.

The phrase "selectively (or specifically) hybridizes to" refers to the binding, duplexing, or hybridizing of a molecule to a particular nucleotide sequence under stringent hybridization conditions when that sequence is present in a mixture (*e.g.,* total cellular or library DNA or RNA, an amplification reaction), such that the binding of the molecule to the particular nucleotide sequence is determinative of the presence of the nucleotide sequence is the mixture.

The phrase "stringent hybridization conditions" refers to conditions under which a probe will hybridize to its target subsequence, typically in a complex mixture of nucleic acids, but to no other sequences. Stringent conditions are sequence-dependent and will be different in different circumstances. Longer sequences hybridize specifically at higher temperatures. An extensive guide to the hybridization of nucleic acids is found in Tijssen, Techniques in Biochemistry and Molecular Biology--Hybridization with Nucleic Probes, "Overview of principles of hybridization and the strategy of nucleic acid assays" (1993). Generally, stringent conditions are selected to be about 5-10°C lower than the thermal melting point (Tₘ) for the specific sequence at a defined ionic strength pH. The Tₘ is the temperature (under defined ionic strength, pH, and nucleic concentration) at which 50% of the probes complementary to the target hybridize to the target sequence at equilibrium (as the target sequences are present in excess, at Tₘ, 50% of the probes are occupied at equilibrium). Stringent conditions will be those in which the salt concentration is less than about 1.0 M sodium ion, typically about 0.01 to 1.0 M sodium ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. For selective or specific hybridization, a positive signal is at least two times background, preferably 10 times background hybridization. Illustrative stringent hybridization conditions can be as following: 50% formamide, 5x SSC, and 1% SDS, incubating at 42°C, or, 5x SSC, 1% SDS, incubating at 65°C, with wash in 0.2x SSC, and 0.1% SDS at 65°C. For PCR, a temperature of about 36°C is typical for low stringency amplification, although annealing temperatures may vary between about 32°C and 48°C depending on primer length. For high stringency PCR amplification, a temperature of about 62°C is typical, although high stringency annealing temperatures can range from about 50°C to about 65°C, depending on the primer length and specificity. Typical cycle conditions for both high and low stringency amplifications include a denaturation phase of 90°C - 95°C for 30 sec - 2 min., an annealing phase lasting 30 sec. - 2 min., and an extension phase of about 72°C for 1 - 2 min. Protocols and guidelines for low and high stringency amplification reactions are provided, e.g., in Innis et al. (1990) PCR Protocols, A Guide to Methods and Applications, Academic Press, Inc. N.Y.).

Nucleic acids that do not hybridize to each other under stringent conditions are still substantially identical if the polypeptides which they encode are substantially identical. This occurs, e.g., when a copy of a nucleic acid is created using the maximum codon degeneracy permitted by the genetic code. In such cases, the nucleic acids typically hybridize under moderately stringent hybridization conditions. Illustrative "moderately stringent hybridization conditions" include a hybridization in a buffer of 40% formamide, 1 M NaCl, 1% SDS at 37°C, and a wash in 1X SSC at 45°C. A positive hybridization is at least twice background. Those of ordinary skill will readily recognize that alternative hybridization and wash conditions can be utilized to provide conditions of similar stringency. Additional guidelines for determining hybridization parameters are provided in numerous reference, e.g., and Current Protocols in Molecular Biology, ed. Ausubel, *et al.*

"Percent identity" can be determined using methods well known in the art, *e.g.,* the BLAST algorithm set to default parameters. An indication that two nucleic acid sequences or polypeptides are substantially identical is that the polypeptide encoded by the first nucleic acid is immunologically cross reactive with the antibodies raised against the polypeptide encoded by the second nucleic acid, as described below. Thus, a polypeptide is typically substantially identical to a second polypeptide, e.g., where the two peptides differ only by conservative substitutions. Another indication that two nucleic acid sequences are substantially identical is that the two molecules or their complements hybridize to each other under stringent conditions, as described below. Yet another indication that two nucleic acid sequences are substantially identical is that the same primers can be used to amplify the sequences.

The phrase "functional effects" in the context of assays for testing compounds that inhibit activity of a FLT3 protein includes the determination of a parameter that is indirectly or directly under the influence of FLT3 protein or nucleic acid, *e.g.,* a functional, physical, or chemical effect, such as the ability to decrease FLT3 kinase activity, decrease cellular proliferation; decrease cellular transformation; decrease growth factor or serum dependence; alter cell surface marker levels, decrease levels of FLT3 mRNA or protein, or otherwise measure FLT3 activity. "Functional effects" include *in vitro, in vivo,* and *ex vivo* activities.

As used herein, "inhibitors" or "antagonists" of FLT3 refer to modulatory molecules or compounds that, *e.g.,* bind to, partially or totally block activity, decrease, prevent, delay activation, inactivate, desensitize, or down regulate the activity or expression of FLT3. Inhibitors can include siRNA or antisense RNA, *e.g.,* siRNA or antisense RNA to target FLT3 nucleic acids or genetically modified versions of FLT3 protein, *e.g.,* versions with altered activity, as well as naturally occurring and synthetic FLT3 antagonists, antibodies, small chemical molecules and the like. FLT3 tyrosine kinase inhibitors are known and include inhibitors such as AC220 and midostaurin. inhibitors for use in the disclosure are known in the art.

In some embodiments, samples or assays comprising FLT3 proteins that are treated with a potential inhibitor are compared to control samples without the inhibitor, to examine the effect on activity. For example, typically, control samples, *e.g.,* AML cells, that have an initial activating mutation and a resistance FLT3 mutation as described herein and that are untreated with inhibitors are assigned a relative protein activity value of 100%. Inhibition of FLT3 is achieved when the activity value relative to the control is changed at least 20%, preferably 50%, more preferably 75-100%, or more.

As used herein, "antibody" includes reference to an immunoglobulin molecule immunologically reactive with a particular antigen, and includes both polyclonal and monoclonal antibodies. The term also includes genetically engineered forms such as chimeric antibodies (*e.g.,* humanized murine antibodies) and heteroconjugate antibodies (*e.g.,* bispecific antibodies). The term "antibody" also includes antigen binding forms of antibodies, including fragments with antigen-binding capability (*e.g*., Fab', F(ab')₂, Fab, Fv and rIgG. *See also,* Pierce Catalog and Handbook, 1994-1995 (Pierce Chemical Co., Rockford, IL). *See also, e.g.,* Kuby, J., Immunology, 3rd Ed., W.H. Freeman & Co., New York (1998). The term also refers to recombinant single chain Fv fragments (scFv). The term antibody also includes bivalent or bispecific molecules, diabodies, triabodies, and tetrabodies.

An antibody immunologically reactive with a particular antigen can be generated by recombinant methods such as selection of libraries of recombinant antibodies in phage or similar vectors, *see, e.g.,* Huse et al., Science 246:1275-1281 (1989); Ward et al., Nature 341:544-546 (1989); and Vaughan et al., Nature Biotech. 14:309-314 (1996), or by immunizing an animal with the antigen or with DNA encoding the antigen.

Typically, an immunoglobulin has a heavy and light chain. Each heavy and light chain contains a constant region and a variable region, (the regions are also known as "domains"). Light and heavy chain variable regions contain four framework" regions interrupted by three hypervariable regions, also called complementarity-determining regions (CDRs).

The term "fully human antibody" refers to an immunoglobulin comprising human hypervariable regions in addition to human framework and constant regions. Such antibodies can be produced using various techniques known in the art.

### Introduction

The present disclosure provides methods, reagents and kits, for detecting AML cells for diagnostic and prognostic uses, and for treating AML patients. The invention is based, in part, upon the discovery that patients that have an initial FLT3 activating mutation, *e.g.,* an ITD mutation, and are treated with AC220 can develop a second mutation that leads to resistance to AC220 and thus, relapse. The resistance mutation occurs typically occur at positions F691, D835, or Y842. In some aspects, the resistance mutation may be at position A848, N841, or D839. In some embodiments, the patient may have resistance mutations at two of positions F691, D835, or Y842. In some embodiments, the initial FLT3 activating mutation may be at Y842 (without an ITD mutation) and the second mutation that leads to resistance is at F691 or D835. Detection of a resistance mutation can be used to identify patients that may relapse, to monitor progression of AML in the patient or efficacy of an AML treatment, and/or to identify patients that are candidates for treatment for a therapeutic alternative to AC220.

### General recombinant methods

This invention relies in part on routine techniques in the field of recombinant genetics, e.g., for methods used in detecting mutations in FLT3, or for the preparation of FLT3 polypeptides and nucleic acids. Basic texts disclosing the general methods of use in this invention include Sambrook & Russell, Molecular Cloning, A Laboratory Manual (3rd Ed, 2001); and Current Protocols in Molecular Biology, Ausubel, 1994-2009, including supplemental updates through April 2010).

### AML patients

In the present invention, the presence of a resistance mutation is analyzed in an AML cell sample from a patient that has been treated with AC220. The patient has a first activating mutation in a FLT3 gene, *e.g.,* an activating in tandem duplication (ITD) mutation in FLT3.

ITD mutations are known in the art. These typically occur within the juxtamembrane domain (see, *e.g.,* Weisberg et al., Oncogene 29:5120-5134, 2010 and references cited therein) and are the most common FLT3 mutation in AML. ITD mutations are a prognostic indicator associated with adverse disease outcome (see, *e.g.,* Thiede et al., Blood 99, 4326-4335, 2002). FLT3-ITD mutations are associated with activation of AKT, the downstream effector of PI3 kinase. In typical instances, the ITD insertion mutations are variable in length, for example, they can be anywhere from 3-400 bp (in-frame) in the juxtamembrane region, but typically there is a supplication of amino-acid residues Y591-Y597, which encodes the switch and zipper regions of the juxtamembrane of FLT3.

In some embodiments, the patient has an initial mutation at Y842 and a resistance mutation at D835 or F691.

Other activating point mutation have been identified in FLT3. Additional activating point mutations have also been identified in a 16 amino acid stretch of the FLT3 juxtamembrane domain and in the tyrosine kinase domain.

In the current invention, the AML patient that has an initial FLT3-activating mutation, e.g., an ITD mutation, has been treated with AC220. AC220 (N-(5-tert-butyl-isoxazol-3-yl)-N'-{4-[7-(2-morpholin-4-yl-ethoxy)imidazo[2,1-b][1,3]benzothiazol-2-yl]phenyl}urea dihydrochloride; also referred to as quizartinib dihydrochloride, Ambit Biosciences, CAS No. 950769-58-1 (free base) and CAS No. 1132827-21-4 (2HCl)) is a small molecule inhibitor that was expressly optimized as a FLT3 inhibitor for the treatment of AML (see, *e.g.,* Chao et al., J. Med. Chem 52:7808-7816, 2009; Zarrinkar, et al., Blood 114:2984-2992, 2009). The present invention provides methods of identifying a patient that has an AC220 drug resistance mutation in FLT3, where the presence of the resistance mutation is indicative of an increased likelihood for relapse compared to an AC220-treated patient that does not have such a FLT3 mutation and/or an increased likelihood for progression of AML.

### Detection of Mutations

In the current invention, the resistance mutation is an amino acid substitution that occurs at F691, D835, or Y842, *e.g.,* D835Y, D835V, D835F, or F691L. In some embodiments, the patient has a substitution at more than one position, *e.g.,* position F691 and position D835. In some aspects, the resistance mutation is an amino acid substitution that occurs at A848, N841, or D839. In some aspects, the resistance mutation is A848P, N841K, F691I, D835Y, D839V, Y842C, or Y842H.

In typical embodiments, nucleic acids from AML cells present in a biological sample from the patient are analyzed for the presence of a sequence mutation at F691, D835, or Y842. In some aspects, the sample is analyzed for the presence of a sequence mutation at A848, N841, or D839. Methods of evaluating the sequence of a particular gene are well known to those of skill in the art, and include, *inter alia,* hybridization and amplification based assays.

In typical aspects, amplification-based assays are employed in methods to detect mutations at a codon that encodes F691, D835, or Y842 of FLT3; or a codon that encodes A848L N841, or D839 of FLT3. In such an assay, the target FLT3 nucleic acid sequence is specifically amplified in an amplification reaction (*e.g.,* Polymerase Chain Reaction, or PCR). Examples of amplification-based assays include RT-PCR methods well known to the skilled artisan *(see, e.g.,* Ausubel *et al., supra*). Detailed protocols for PCR of DNA and RNA, including quantitative amplification methods,are known (*see,* e.g., Innis et al. (1990) PCR Protocols, A Guide to Methods and Applications, Academic Press, Inc. N.Y.; and Ausubel and Russell & Sambrook, *both supra*). The known nucleic acid sequences for FLT3 are sufficient to enable one of skill to routinely select primers to specifically amplify any portion of the gene so that the desired region of FLT3 is targeted. Suitable primers for amplification of specific sequences can be designed using principles well known in the art *(see, e.g.,* Dieffenfach & Dveksler, PCR Primer: A Laboratory Manual (1995)).

Other suitable amplification methods include, but are not limited to, ligase chain reaction (LCR) (*see,* Wu and Wallace (1989) Genomics 4: 560, Landegren et al. (1988) Science 241:1077, and Barringer et al. (1990) Gene 89: 117), transcription amplification (Kwoh et al. (1989) Proc. Natl. Acad. Sci. USA 86: 1173), self-sustained sequence replication (Guatelli et al. (1990) Proc. Nat. Acad. Sci. USA 87: 1874), dot PCR, and linker adapter PCR, etc.

In some embodiments, the presence of a resistance mutation at F691, D835, or Y842 allele can be conveniently determined using DNA sequencing, including sequencing by synthesis methods, sequencing by ligation, and sequencing by expansion methodologies. Technologies include pyrosequencing, ion semiconductor sequencing, nanopore sequencing, single molecule sequence, *e.g.* real time single molecule sequencing technology, or other sequencing methods. In some embodiments, single molecule sequencing is employed (*e.g.,* the True Single Molecule Sequencing (tSMS™) sequencing platform (Helicos BioSciences Corporation); or Real Time Single Molecule Sequencing (SMRT™) sequencing platform (Pacific Biosciences Incorporated)).

In some aspects, a resistance mutation at a codon that encodes F691, D835, or Y842; or that encodes A848, N841, or D839, is determined by hybridization of a sample DNA or RNA to a probe that specifically hybridizes to a FLT3 sequence. The probes used in such applications specifically hybridize to the region of the FLT3 sequence harboring the mutation. Preferred probes are sufficiently long, *e.g.,* from about 10, 15, or 20 nucleotides to about 50 or more nucleotides, so as to specifically hybridize with the target nucleic acid(s) under stringent conditions.

Any of a number hybridization-based assays can also be used to detect a sequence mutation at a codon that encodes F691, D835, or Y842; or A848, N841, or D839 in nucleic acids obtained from an AML cell sample. In some embodiments, DNA or RNA obtained from the AML cell sample can be evaluated using known techniques such as allele-specific oligonucleotide hybridization, which relies on distinguishing a mutant position in a nucleic acid from a normal position in a nucleic acid sequence using an oligonucleotide that specifically hybridizes to the mutant or normal nucleic acid sequence. This method typically employs short oligonucleotides, *e.g.,* 15-20 nucleotides, in length, that are designed to differentially hybridize to the normal or mutant allele. Guidance for designing such probes is available in the art. The presence of a mutant allele is determined by measuring the amount of allele-specific oligonucleotide that hybridizes to the sample.

In other embodiments, the presence of a normal or mutant FLT3 nucleic acid can be detected using allele-specific amplification or primer extension methods. These reactions typically involve use of primers that are designed to specifically target a normal or mutant allele via a mismatch at the 3' end of a primer. The presence of a mismatch affects the ability of a polymerase to extend a primer when the polymerase lacks error-correcting activity. The amount of amplified product can be determined using a probe or by directly measuring the amount of DNA present in the reaction.

Detection of levels of nucleic acids in an AML cell sample that have a mutation at a codon encoding F691, D835, or Y842, or A848, N841, or D839 can also be performed using a quantitative assay such as a 5'-nuclease activity (also referred to as a "TaqMan®" assay), *e.g.,* as described in U.S. Pat. Nos. 5,210,015; 5,487,972; and 5,804,375; and Holland et al., 1988, Proc. Natl. Acad. Sci. USA 88:7276-7280. In such an assay, labeled detection probes that hybridize within the amplified region are added during the amplification reaction. In some embodiments, the hybridization probe can be an allele-specific probe that discriminates a normal or mutant allele. Alternatively, the method can be performed using an allele-specific primer and a labeled probe that binds to amplified product.

Other detection methods include single-stranded conformational analysis, amplicon melting analysis, or methods based on mass spectrometry. Mass spectrometry takes advantage of the unique mass of each of the four nucleotides of DNA. The allele can be unambiguously genotyped by mass spectrometry by measuring the differences in the mass of nucleic acids having alternative FLT3 alleles. MALDI-TOF (Matrix Assisted Laser Desorption Ionization - Time of Flight) mass spectrometry technology is preferred for extremely precise determinations of molecular mass, such as single nucleotide mutations. Preferred mass spectrometry-based methods of single nucleotide mutation assays include primer extension assays, which can also be utilized in combination with other approaches, such as traditional gel-based formats and microarrays.

### Detection of polypeptide sequences comprising a resistance mutation associated with treatment with AC220

FLT3 mutations may also be detected by detecting mutant protein. For example, detection of FLT3 proteins that have a mutation at F691, D835 or Y842 can be used for diagnostic purposes or in screening assays. In some embodiments, the presence of a mutant FLT3 polypeptide in a sample is conveniently determined using immunological assays using reagents, *e.g.,* an antibody, that specifically detects mutant FLT3 mutations. The detection and/or quantification of FLT3 proteins having mutations at F691, D835, or Y842 can be accomplished using any of a number of well recognized immunological binding assays. A general overview of the applicable technology can be found in Harlow & Lane, Antibodies: A Laboratory Manual (1988) and Harlow & Lane, Using Antibodies (1999). Other resources include see also Methods in Cell Biology: Antibodies in Cell Biology, volume 37 (Asai, ed. 1993); Basic and Clinical Immunology (Stites & Terr, eds., 7th ed. 1991, and Current Protocols in Immunology (Coligan, et al. Eds, John C. Wiley, 1999-present). Immunological binding assays can use either polyclonal or monoclonal antibodies. In some embodiments, antibodies that specifically detect mutant FLT3 molecules may be employed.

Commonly used assays include noncompetitive assays (e.g., sandwich assays) and competitive assays. Commonly used assay formats include immunoblots, which are used to detect and quantify the presence of protein in a sample. Other assay formats include liposome immunoassays (LIA), which use liposomes designed to bind specific molecules (e.g., antibodies) and release encapsulated reagents or markers, which are then detected according to standard techniques (see Monroe et al., Amer. Clin. Prod. Rev. 5:34-41 (1986)).

FLT3, or a fragment thereof, *e.g.,* the portion of the peptide containing the activating sequence mutation, may be used to produce antibodies specifically reactive with FLT3 using techniques known in the art *(see, e.g.,* Coligan; Harlow & Lane, both *supra*). Such techniques include antibody preparation by selection of antibodies from libraries of recombinant antibodies in phage or similar vectors, as well as preparation of polyclonal and monoclonal antibodies by immunizing rabbits or mice (see, e.g., Huse et al., Science 246:1275-1281 (1989); Ward et al., Nature 341:544-546 (1989)). Such antibodies can be used for diagnostic or prognostic applications.

In some aspects, a FLT3 antibody may be used for therapeutic applications. For example, in some aspects, such an antibody may used to reduce or eliminate a biological function of a FLT3 having an activating mutation at F691, D835, or Y842. Typically, antibodies for therapeutic use are humanized or human antibodies. Such antibodies can be obtained using known techniques.

As appreciated by one of skill in the art, FLT3 activity can be detected to evaluate expression levels of FLT3 proteins having an activating mutation at F691, D835, or Y842 or for identifying inhibitors of activity. The activity can be assessed using a variety of *in vitro* and *in vivo* assays, including protein kinase activity. In some aspects FLT3 activity can be evaluated using additional endpoints, such as those associated with PI3 kinase activity, or transformation.

### Diagnostic/prognostic uses

FLT3 nucleic acid and polypeptide sequences can be evaluated for diagnosis or prognosis of AML in a patient treated with ACC that has an initial FLT 3 activating mutation, *e.g.,* an ITD mutation. For example, as described above, the sequence of FLT3 in an AML cell sample from a patient can be determined, wherein a mutation in a codon that encodes F691, D835, or Y842 indicates the presence or the likelihood that the patient will have a relapse. In some aspects, the patient treated with ACC has an initial FLT3 activating mutation at Y842. As a further example, the sequence of FLT3 in an AML cell sample from the patient can be determined wherein a mutation at a codon that encodes F691 or D835 indicates the presence or the likelihood that the patient will have a relapse.

The methods of the present invention can be used to determine the optimal course of treatment in a patient with cancer. For example, the presence of a resistance mutation in a codon encoding F691, D835, or Y842, or in a codon encoding A848, N841, or D839, may indicate that an alternate therapy to AC220, such as a therapy that targets a downstream pathway regulated by FLT3 will be beneficial to those patients. In addition, a correlation can be readily established between the number of AML cells having the resistance mutation, and the relative efficacy of AC220 by correlating the number of AML cells having the mutation with the efficacy of the treatment.

Such methods can be used in conjunction with additional diagnostic methods, e.g., detection of other AML relapse indicators.

Any biological sample AML cells can be evaluated to determine the presence of a resistance mutation at F691, D835, or Y842, or at A848, N841, or D839. Typically, a blood or bone marrow sample is evaluated, but a sample obtained from a metastatic site, *e.g.,* from spinal chord or brain, may also be employed to analyze the FLT in AML cells to determine whether a second activating mutation is present.

In some aspects, the methods of the disclosure involve recording the presence or absence of a resistance mutation at F691, D835, or Y842, or at A848, N841, or D839, in AML cells in patients who have been treated with AC220. This information may be stored in a computer readable form. Such a computer system typically comprises major subsystems such as a central processor, a system memory (typically RAM), an input/output (I/O) controller, an external device such as a display screen via a display adapter, serial ports, a keyboard, a fixed disk drive via a storage interface and the like. Many other devices can be connected, such as a network interface connected via a serial port.

The computer system also be linked to a network, comprising a plurality of computing devices linked via a data link, such as an Ethernet cable (coax or 10BaseT), telephone line, ISDN line, wireless network, optical fiber, or other suitable signal transmission medium, whereby at least one network device (e.g., computer, disk array, etc.) comprises a pattern of magnetic domains (e.g., magnetic disk) and/or charge domains (e.g., an array of DRAM cells) composing a bit pattern encoding data acquired from an assay of the invention.

### Inhibitors or modulators of FLT3

In another aspect, this disclosure includes methods of inhibiting the proliferation of AML cells from patients treated with ACC220 that have an initial activating mutation, *e.g.,* ITD mutations, and a second FLT3 mutation that leads to resistance (a substitution at position F691, D835, or Y842) where the method comprises administering a further FLT3 inhibitor to the patient that has the resistance mutation. Inhibitors can include inhibitors of downstream FLT3 effectors, *e.g.,* PI3 kinase inhibitors, or other agents. In some aspects, the inhibitor may be an alternative tyrosine kinase inhibitor, *e.g.,* PLX3397 (Plexxikon Inc, Berkeley, CA), ponatinib (Ariad Pharmaceuticals), G749 (Genosco, Cambridge, MA), or crenolanib (AROG Pharmaceuticals). In some aspects, *e.g.,* where the mutation is at F691, the inhibitor may be ponatinib.

Other inhibitors include antibodies, peptides, nucleic acids, *e.g.,* siRNA, and the like. As used herein, a FLT3 inhibitor can be a molecule that modulates FLT3 nucleic acid expression and/or FLT3 protein activity, or in some aspects, downstream pathways regulated by FLT3. In some aspects, a FLT3 inhibitor is an inhibitory RNA molecule that targets *FLT3* nucleic acid sequences.

The ability to inhibit FLT3 can be evaluated using appropriate assays, *e.g.,* by assaying activity, *e.g.,* kinase activity and comparing the amount of activity to controls that are not treated with the inhibitor.

In another aspect, mRNA and/or protein expression levels can be measured to assess the effects of a test compound on FLT3 expression levels. A host cell expressing FLT3 is contacted with a test compound for a sufficient time to effect any interactions, and then the level of mRNA or protein is measured. The amount of time to effect such interactions may be empirically determined, such as by running a time course and measuring the level of expression as a function of time. The amount of expression may be measured by using any method known to those of skill in the art to be suitable.

The amount of expression is then compared to the amount of expression in the absence of the test compound. A substantially identical cell may be derived from the same cells from which the recombinant cell was prepared but which had not been modified by introduction of heterologous DNA. A difference in the amount of expression indicates that the test compound has in some manner altered FLT3 levels.

In some assays to identify FLT3 inhibitors, samples that are treated with a potential inhibitor are compared to control samples to determine the extent of modulation. Control samples without the mutation and untreated with candidate inhibitors are assigned a relative activity value of 100. Inhibition of FLT3 is achieved when the activity value relative to the control is about 80%, optionally 50%, optionally 25-0%.

FLT3 inhibitors can be any small chemical compound, or a biological entity, *e.g.,* a macromolecule such as a protein, sugar, nucleic acid or lipid.

In some aspects, FLT3 inhibitors that are evaluated to treat AC220-refractory AML are small molecules that have a molecular weight of less than 1,500 daltons, and in some cases less than 1,000, 800, 600, 500, or 400 daltons. The relatively small size of the agents can be desirable because smaller molecules have a higher likelihood of having physiochemical properties compatible with good pharmacokinetic characteristics, including oral absorption than agents with higher molecular weight. For example, agents less likely to be successful as drugs based on permeability and solubility were described by Lipinski *et al.* as follows: having more than 5 H-bond donors (expressed as the sum of OHs and NHs); having a molecular weight over 500; having a LogP over 5 (or MLogP over 4.15); and/or having more than 10 H-bond acceptors (expressed as the sum of Ns and Os). *See, e.g.,* Lipinski et al. Adv Drug Delivery Res 23:3-25 (1997). Compound classes that are substrates for biological transporters are typically exceptions to the rule.

In some aspects, nucleic acid inhibitors may be used to inhibit FLT3 in a patient having AML cells with an initial FLT3 activating mutation, *e.g.,* an ITD mutation, that is identified as having a second mutation at a codon encoding D835Y, F691, or Y842. For example, a nucleotide sequence such as an siRNA and/or antisense oligonucleotides to block transcription or translation of FLT3 mRNA, either by inducing degradation of the mRNA with a siRNA or by masking the mRNA with an antisense nucleic acid can be employed.

An "siRNA" or "RNAi" refers to a nucleic acid that forms a double stranded RNA, which double stranded RNA has the ability to reduce or inhibit expression of a gene or target gene when the siRNA expressed in the same cell as the gene or target gene. "siRNA" thus refers to the double stranded RNA formed by the complementary strands. The complementary portions of the siRNA that hybridize to form the double stranded molecule typically have substantial or complete identity. The sequence of the siRNA can correspond to the full length target gene, or a subsequence thereof. Typically, the siRNA is at least about 15-50 nucleotides in length (*e.g.,* each complementary sequence of the double stranded siRNA is 15-50 nucleotides in length, and the double stranded siRNA is about 15-50 base pairs in length, preferably about preferably about 20-30 base nucleotides, preferably about 20-25 nucleotides in length, e.g., 20, 21, 22, 23, 24, 25, 26, 27, 28, 29, or 30 nucleotides in length.

"Silencing" or "downregulation" refers to a detectable decrease of transcription and/or translation of a target sequence, *i.e.,* the sequence targeted by the siRNA, or a decrease in the amount or activity of the target sequence or protein in comparison to the normal level that is detected in the absence of the interfering RNA or other nucleic acid sequence. A detectable decrease can be as small as 5 % or 10 %, or as great as 80 %, 90 % or 100 %. More typically, a detectable decrease ranges from 20 %, 30 %, 40 %, 50 %, 60 %, or 70 %.

A DNA molecule that transcribes dsRNA or siRNA (for instance, as a hairpin duplex) also provides RNAi. For example, dsRNA oligonucleotides that specifically hybridize to a *FLT3* nucleic acid sequenc can be used therapeutically.

Antisense oligonucleotides that specifically hybridize to FLT3 nucleic acid sequences can also be used to silence the transcription and/or translation of FLT3 and thus treat AML. Methods of designing antisense nucleic acids (either DNA or RNA molecules) are well known in the art. Antisense nucleic acids may comprise naturally occurring nucleotides or modified nucleotides such as, *e.g.,* phosphorothioate, methylphosphonate, and -anomeric sugar-phosphate, backbone-modified nucleotides.

The ability of an inhibitor to modulate the expression of FLT3 can be evaluated using known methods. Such methods generally involve conducting cell-based assays in which test compounds are contacted with one or more cells expressing FLT3 and then detecting a decrease in expression (either transcript or translation product).

### Treatment and administration of pharmaceutical compositions

Inhibitors of FLT3 can be administered to a patient for the treatment of an AML that has an initial activating FLT3 mutation and a resistance mutation at position F691, D835, or Y842; and is refractory to AC220 treatment. The inhibitors are administered in any suitable manner, optionally with pharmaceutically acceptable carriers. Protocols for the administration of inhibitors are known and can be further optimized for AML patients based on principles known in the pharmacological arts (see, *e.g*., Remington: The Science and Practice of Pharmacy, 21st Edition, Philadelphia, PA. Lippincott Williams & Wilkins, 2005).

A FLT3 inhibitor can be administered to a patient at therapeutically effective dose to prevent, treat, or control AML. The compounds are administered to a patient in an amount sufficient to elicit an effective therapeutic response in the patient. An effective therapeutic response is a response that at least partially arrests or slows the symptoms or complications of AML. An amount adequate to accomplish this is defined as "therapeutically effective dose." The dose will be determined by the efficacy of the particular FLT3 inhibitor employed and the condition of the subject, as well as the body weight or surface area of the area to be treated. The size of the dose also will be determined by the existence, nature, and extent of any adverse effects that accompany the administration of a particular compound in a particular subject.

FLT3 nucleic acid inhibitors, *e.g.,* siRNA, can be delivered to the subject using any means known in the art, including by injection of the siRNA. In addition, polynucleotide inhibitors can be delivered using a recombinant expression vector (*e.g.,* a viral vector based on an adenovirus, a herpes virus, a vaccinia virus, or a retrovirus;) or a colloidal dispersion system (*e.g.,* liposomes).

A treatment that targets FLT3 can be administered with other AML therapeutics, either concurrently or before or after treatment with another AML therapeutic agent.

### Kits for Use in Diagnostic and/or Prognostic Applications

The disclosure also provides kits for diagnostic or therapeutic applications. For diagnostic/prognostic applications, such kits may include any or all of the following: assay reagents, buffers, FLT3 probes, primers, antibodies, or the like that can be used to identify the presence of a mutation at the codon for D835, F691, or Y842. In some aspects, the probes, primers or other reagents may detect a D835Y, D835V, or D835F mutation. In some aspects, the probes or primers may detect a F691L mutation. In some aspects, the probes, primers or other reagents may detect a mutation at the codon for A848, N841, or D839. In some aspects, the probes, primers or other reagents may detect an A848P, N841K, F691I, D839V, Y842C, or Y842H mutation.

In addition, the kits may include instructional materials containing directions (i.e., protocols) for the practice of the methods of this invention. While the instructional materials typically comprise written or printed materials they are not limited to such. Any medium capable of storing such instructions and communicating them to an end user is contemplated by this disclosure. Such media include, but are not limited to electronic storage media (e.g., magnetic discs, tapes, cartridges, chips), optical media (e.g., CD ROM), and the like. Such media may include addresses to internet sites that provide such instructional materials.

The following examples are provided by way of illustration only and not by way of limitation. Those of skill in the art will readily recognize a variety of noncritical parameters that could be changed or modified to yield essentially similar results.

### EXAMPLES

In these examples, we sought, in part, to use the clinical activity of AC220 as a tool to properly define FLT3-ITD as a "driver" or "passenger" mutation in human AML. Using a previously validated *in vitro* saturation mutagenesis assay¹⁴, we identified AC220 resistance-conferring mutations at four residues in FLT3-ITD (Fig. 1a). These mutations, when recreated and introduced into Ba/F3 cells, conferred growth factor independence, suggesting retention of pathologically activated kinase activity. Mutations at three of these amino acid positions yielded FLT3-ITD isoforms with high degrees of resistance to AC220 *in vitro* (Fig. 1b). These residues consist of the "gatekeeper" residue (F691) and two residues within the activation loop (D835, Y842). Although E608K mutations were repeatedly isolated in our screen, for unclear reasons, this substitution failed to confer significant resistance when reintroduced into Ba/F3 cells and was therefore not further characterized. Mutations at F691, D835 and Y842 demonstrated clear evidence of biochemical resistance to AC220 relative to FLT3-ITD in a cell-based assay (Fig. 1c).

We next assessed for the presence of drug-resistant FLT3-ITD kinase domain mutations in paired pretreatment and relapse samples obtained from nine FLT3-ITD+ AML patients who initially achieved morphologic clearance of bone marrow blasts to less than 5% on AC220 in the exploratory part of the ongoing phase II study, but subsequently relapsed despite continuing therapy. In every case, subcloning and sequencing of individual FLT3-ITD alleles as previously described¹⁵ revealed the presence of mutations occurring at one or more of the three critical residues identified in our *in vitro* screen at the time of relapse (Table 1). None of these mutations were detected in the pretreatment samples of these patients (data not shown). The activation loop mutation D835Y was detected in four of these nine cases, D835V in two, and the gatekeeper mutation F691L was identified in three. One patient sample (1011-007) appeared to have evolved polyclonal resistance, with both F691L and D835V mutations detected on separate FLT3-ITD sequences. Additionally, one novel mutation, D835F, was identified in a single patient. This mutation conferred *in vitro* resistance to AC220 and cross-resistance to sorafenib (Figure 3), and was most likely not recovered in our saturation mutagenesis screen because it represents a two-nucleotide substitution. Collectively, these findings suggest that clinical response and relapse in each of these nine patients mechanistically involved FLT3 kinase activity, and further identified FLT3-ITD alleles as "driver" mutations and valid therapeutic targets in human AML.

Two additional patients who had a deep response to AC220 and had intial ITD mutations were also evaluated. These two patients also had resistance-conferring mutations at the time of relapse. One of the patients had a D835V mutation and the other patient had a D835Y mutation.

To more precisely assess for resistance-conferring mutations at relapse, we utilized a recently described single molecule real-time (SMRT™; Pacific Biosciences, Menlo Park, CA) sequencing platform, which can provide reads of sufficient length to enable focused interrogation of the kinase domain of FLT3-ITD alleles^{16,17}. With this assay, hundreds of reads (range 380-3532) of greater than 1 kb were reliably obtained. Attention was focused on the amino acid residues identified in the *in vitro* screen for AC220 resistance-conferring mutations. Analysis of a normal control sample revealed the presence of base substitutions at these residues at a frequency of less than two percent. Analyses of pretreatment and relapse samples from four AML patients confirmed the presence of resistance-conferring FLT3-ITD kinase domain mutations at relapse (Table 2). Consistent with the results described above, mutations at E608 and Y842 were not detected. The frequency of mutation representation at relapse ranged from 3.6% (D835V in patient 1009-003) to 55.7% (D835Y in patient 1011-007). The presence of polyclonal resistance in patient 1011-007 was confirmed, and also noted in a second of these four cases (1009-003). The evolution of polyclonal resistance due to FLT3-ITD kinase domain mutations is further suggestive of a central dependence upon FLT3-ITD signaling in the leukemic clone in a subset of AML patients.

Relapse occurred relatively rapidly in some patients; nonetheless, most mutations were not convincingly detectable prior to treatment when assessed by SMRT™ sequencing.

The five substitutions that conferred a high degree of resistance to AC220 *in vitro* were assessed for sensitivity to sorafenib. Although FLT3-ITD/D835V has been previously reported to retain sensitivity to sorafenib in vitro¹⁸, we found that all AC220-resistant mutations conferred substantial cross-resistance to sorafenib in cell-based growth (Figure 4A) and biochemical assays (Figure 4B). Indeed, the degree of resistance to sorafenib of these mutations relative to FLT3-ITD alone was in general agreement with the degree of resistance conferred to AC220 (Figure 4C).

Modeling of FLT3-AC220 interactions was performed to gain insights into the structural sequelae of AC220 resistance-conferring mutations identified in our studies (Fig. 2a). The crystal structure of the FLT3 kinase domain has been previously determined in an inactive conformation¹⁹ that closely resembles the inactive conformations of c-ABL²⁰, c-KIT²¹, and insulin receptor tyrosine kinase²². In this crystal structure, the activation loop is folded back onto the ATP-binding cleft (loop-in conformation), and blocks ATP entry and substrate loading. In addition, the Asp-Phe-Gly (DFG) motif adopts the DFG-out conformation that is not capable of coordinating magnesium iron-ATP binding. The activation of FLT3 would require flipping of the DFG motif and the unfolding of the activation loop, as observed in c-Abl²³ and insulin receptor kinase²⁴. The molecular docking study strongly suggested that AC220 specifically targets the DFG-out, inactive FLT3 conformation, and provides a potential structural basis for AC220 resistance-conferring mutations at D835, Y842 and F691. In the docked AC220-FLT3 complex structure, an AC220 phenol-ring moiety forms a close T-shaped *π*-*π* stacking contact with F830 in the DFG motif (Fig. 2b). This interaction would not be possible in the DFG-in, active kinase conformation. The gatekeeper residue F691 forms a parallel *π*-*π* stacking contact with AC220 benzo-imidazol-thiazol moiety, further stabilizing the complex. Substitutions at F691 with non-aromatic residues such as leucine will presumably decrease the binding affinity between AC220 and FLT3. Residues D835 and Y842 stabilize the folded activation loop by forming hydrogen-bonds with a main chain amide and D811, respectively (Fig. 2c). Mutations at either residue are predicted to destabilize the folded activation loop, and ease the conformation transition from the inactive state to the active state. This effect will likely hinder the inhibitory function of AC220, since the drug specifically targets the inactive kinase conformation. The ability to retain inhibitory activity against activation loop substitutions at positions D835 and Y842 will likely require a FLT3 kinase inhibitor that is capable of effectively binding to the active, DFG-in conformation of the kinase.

Substitutions at "gatekeeper" residues (BCR-ABL/T315I¹⁵, EGFR/T790M²⁵, KIT/T670I²⁶, EML4-ALK/L1196M²⁷) such as FLT3-ITD/F691 identified here, are well-documented causes of resistance to kinase inhibitors. Analogs of the FLT3-ITD/D835V activation loop mutation have proven problematic for a number of kinase inhibitors: KIT/D816V, an activating mutation that is highly associated with systemic mastocytosis and occasionally with gastrointestinal stromal tumors and AML, confers a high degree of resistance to imatinib and other KIT inhibitors²⁸. Our data, although derived from a small cohort of patients that will need to be validated in larger studies, suggest that substitutions at F691 and D835 in FLT3-ITD will pose substantial barriers to longer-term disease control in AML patients treated with either AC220 or sorafenib. Gatekeeper and activation loop mutations in FLT3-ITD identified herein therefore represent high-value targets for novel FLT3 inhibitor development strategies.

Compelling data suggest that activating FLT3 mutations are acquired relatively late during leukemogenesis in a pre-established clone^{1,4,5}, and alone are insufficient to cause acute leukemia in pre-clinical models^{2,3}. Recent evidence suggests that the molecular heterogeneity of individual leukemias can be substantial, and can occur in both branching and linear fashions early during leukemogenesis, including at the leukemia-initiating or "leukemic stem" cell level^{29,30}. In light of these observations and the cumulative clinical experience with prior FLT3 inhibitors, it is unexpected that complete remission in FLT3-ITD+ AML could commonly be achieved through FLT3 inhibition. However, our demonstration that acquired resistance to clinically effective FLT3 inhibitor therapy is frequently associated with restoration of FLT3-ITD activity through acquisition of drug-resistant kinase domain mutations in FLT3-ITD validates FLT3-ITD as a therapeutic target in human AML. Collectively, our data are consistent with acquisition of FLT3-ITD and drug-resistant FLT3 kinase domain mutations in a leukemia-initiating cell population, although formal transplantation studies in mice are needed to definitively address this issue.

Our findings suggest that FLT3-ITD is capable of conferring a state of "oncogene addiction", whereby cellular survival pathways associated with normal or precancerous cells can become hijacked, leading to a state of exquisite reliance upon key signaling molecules that can be exploited with targeted therapeutics. This work supports the exploration of therapeutic strategies targeting select activating mutations in other signaling molecules that are believed to be acquired relatively late in disease evolution, such as mutations in *JAK2³¹* or *RAS*, with agents capable of achieving clinically meaningful target inhibition. Further studies will be required to identify mechanisms of drug resistance that may circumvent reliance on activated FLT3 by activation of downstream pathways, as has been described with other TKIs^{32,33}. To that end, translational studies employing detailed molecular analyses of genetic variation in primary samples, obtained from AML patients treated with clinically effective targeted therapeutics, promise to further inform mechanisms of drug resistance, strategies for future drug development, and models of disease evolution.

Additional experiments using methodology described herein identified additional resistance mutations at position D835 and F691 and mutations at positions A848, N841, and D839. These resistance mutations include A848P, N841K, F691I, D835Y, and D839V.

### MATERIALS AND METHODS

**DNA Constructs, Mutagenesis and Resistance Screen.** FLT3-ITD cDNA cloned from the MV4;11 cell line (ITD: residues 591-601) into the *HpaI* site of the pMSCV puro retroviral vector (Clontech) was the kind gift of Ambit Biosciences and was used as a template for mutagenesis. We used a modified strategy for random mutagenesis previously described by others¹⁴. Briefly, 1 µg of MSCV FLT3-ITD was used to transform the DNA-repair-deficient *Escherichia coli* strain XL-1 Red (Stratagene) and plated on 20 ampicillin-agar bacterial plates. After incubation for 36 h, colonies were collected by scraping, and plasmid DNA was purified by using a plasmid MAXI kit (Qiagen). Subsequently, mutagenized FLT3 ITD plasmid stock and Ecopack packaging plasmid were cotransfected into 293T cells grown in DMEM (Invitrogen) containing 10% FCS (Omega Scientific) using Lipofectamine 2000 (Invitrogen) per manufacturer's protocol. Viral supernatants were collected at 48 h, purified using a 0.44 µm vacuum filter, and used to infect Ba/F3 cells at a 1:100 to 1:300 dilution of viral supernatant to fresh RPMI 1640 (Invitrogen) supplemented with 10% FCS. Alternatively, viral supernatant was aliquoted and frozen. Thawed supernatant was used to infect Ba/F3 cells at a 1:50 dilution. Viral supernatant was diluted with the goal of minimizing multiplicity of infection. For infection, 1-2 × 10⁶ Ba/F3 cells was resuspended in 3 ml of the diluted viral stock supplemented with recombinant mouse IL-3 (Invitrogen), and 4 µg/ml polybrene, plated in each well of a 12-well tissue culture dish and centrifuged at 1,500 RCF in a Beckman Coulter Allegra 6KR centrifuge with a microplate carrier for 90 min at 34°C. Centrifuged cells were subsequently transferred to a 37°C incubator overnight. Infected Ba/F3 cells were washed twice with media to remove IL-3 and plated in 3 ml of RPMI medium 1640 at 5 × 10⁵ cells per well of a six-well dish supplemented with 20% FCS and 1.2% Bacto-agar with 20nM AC220 (kind gift of Ambit Biosciences). After 10-21 days, visible colonies were plucked from agar and expanded in the presence of drug (20nM AC220).

**Sequencing and Alignments.** Expanded colonies were harvested 7-14 days after isolation from agar, and whole genomic DNA was isolated using the QIAamp kit (Qiagen). FLT3 kinase domain was amplified by PCR from whole genomic DNA by using TopTaq DNA polymerase (Qiagen). The primers TK1F (5'-TGCTGTGCATACAATTCCCTTGGC-3') and TK2R (5'-TCTCTGCTGAAAGGTCGCCTGTTT-3') were used for kinase domain amplification and subsequent bidirectional sequencing was performed using these primers in addition to TK1R (5'-AGTCCTCCTCTTCTTCCAGCCTTT-3') and TK2F (5' GAGAGGCACTCATGTCAGAACTCA-3'). Alignments to the wild type FLT3-ITD sequence were performed using Sequencher software (Gene Codes Corporation).

**Generation of Mutants.** Mutants isolated in the screen were engineered into pMSCV puro FLT3-ITD by using the QuikChange mutagenesis kit (Stratagene). In all cases, individual point mutants were confirmed by sequence analysis.

**Cell-Viability Assay.** Stable Ba/F3 lines were generated by using retroviral spinfection with the appropriate mutated plasmid as outlined above, with the exception of the exclusion of polybrene. At 48 h post-infection, puromycin was added to infected cells at a concentration of 4 µg/mL. Cells were selected in the presence of puromycin for 7-10 days and subsequently IL-3 was washed twice from the cells with media and cells were selected in RPMI medium 1640 + 10% FCS in the absence of IL-3. Exponentially growing Ba/F3 cells (5 × 10⁴) were plated in each well of a 24-well dish with 1 ml of RPMI 1 640 + 10% FCS containing the appropriate concentration of drug as indicated in triplicate. Cells were allowed to expand for 2 days and were counted by using a Vi-cell XR automated cell viability analyzer (Beckman Coulter). The mean number of viable cells at varying concentrations of drug was normalized to the median number of viable cells in the no-drug sample for each mutant. Error bars represent the standard deviation. Numerical IC₅₀ values were generated using non-linear best-fit regression analysis using Prism 5 software (GraphPad).

**Immunoblotting.** Exponentially growing Ba/F3 cells stably expressing each mutant along with a WT FLT3-ITD control were plated in RPMI medium 1640 + 10% FCS supplemented with kinase inhibitor at the indicated concentration. After a 90-minute incubation, the cells were washed in phosphate buffered saline (PBS) and lysed in Cell Extraction Buffer (Invitrogen) supplemented with protease and phosphatase inhibitors. The lysate was clarified by centrifugation and quantitated by BCA assay (Thermo Scientific). Protein was subjected to sodium dodecylsulfate polyacrylamide electrophoresis and transferred to nitrocellulose membranes. Immunoblotting was performed using anti-phospho-FLT3 (Cell Signaling) and anti-FLT3 S18 antibody (Santa Cruz Biotechnology).

**Patients and FLT3 kinase domain sequencing analysis.** Nine cases of acquired resistance to AC220 were analyzed. Patients were enrolled on the Phase II clinical trial of AC220 in relapsed or refractory AML at UCSF, University of Pennsylvania, Johns Hopkins or MD Anderson Cancer Center. Details of the clinical trials and results are reported elsewhere¹³. All patients were FLT3-ITD positive at enrollment. Samples were collected pretreatment and at the time of disease progression. Only patients who had achieved morphologic clearance of bone marrow blasts to less than 5% at best response are included in this analysis. All patients gave informed consent according to the Declaration of Helsinki to participate both in the clinical trials and for collection of samples.

For sequencing, frozen Ficoll-purified mononuclear cells obtained from blood or bone marrow were lysed in Trizol (Invitrogen) and RNA was isolated according to manufacturer protocol. cDNA was synthesized using Superscript II (Invitrogen) per manufacturer's protocol. The *FLT3* kinase domain and adjacent juxtamembrane domain were PCR amplified from cDNA using primer TK1F and TK2R as above. PCR products were cloned using TOPO TA cloning (Invitrogen) and transformed into competent E. coli. Individual colonies were plucked, expanded in liquid culture overnight and plasmid DNA for sequencing was isolated using the QIAprep Spin Miniprep kit (Qiagen). Each colony was considered representative of a single mRNA. To minimize contamination from PCR artifact, we sequenced at least 10 and up to 24 *FLT3*-ITD containing clones from each sample and required that mutations to be found in >15% of clones. The primers TK1F, TK2R, TK2F and TK2R were used for bidirectional sequencing as above. Alignments the wild type FLT3 sequence were performed using Sequencher software (Gene Codes Corporation).

### Sample Preparation and Sequencing

PCR product containing the *FLT3* kinase domain was generated from patient cDNA as described above using high fidelity DNA polymerase. We prepared PCR products for Pacific Biosciences sequencing¹⁷ using standard commercial kits and reagents as shown on the Pacific Biosciences website following the manufacturer's instructions. PCR products input amounts ranged from 0.3 to 3 micrograms, and we prepared SMRTBell libraries¹⁶ on the full PCR products without any fragmentation. We sequenced all samples on a Pacific Biosciences RS instrument and recorded sequence for 45 minutes.

### Computational Analysis of FLT3 Mutations

We obtained a sample from a healthy individual with no cancer history, isolated RNA, made cDNA, amplified the *FLT3* KD, and sequenced following a protocol identical to that used on the AML samples, except that we recorded sequence for a full two hours. We then used the sequence from this healthy individual as a control for all process steps between sample acquisition and sequencing. We first identified the individual ITD sequence for each sample by identifying each subread¹⁶. After identification of all subreads, subreads were clustered by multiple sequence alignments and the consensus sequence generated for each cluster. We used Tandem Repeats Finder (TRF)³⁴ to identify the ITD sequence. We found that each sample had only one major ITD as expected. To unambiguously determine whether a read was ITD- or ITD+, we used only the subreads that included at least the region from the 50-bp 5'-end upstream to 50-bp 3'-end downstream sequence of the ITD region in the analysis. This allowed us to determine the number of sequences containing the ITD more accurately despite potential insertion and deletion error from the single molecule sequencing. An example of the length distribution of the ITD regions is shown in Figure 5. Two distinct peaks allowed us to identify ITD-/ITD+ subreads unambiguously. We then passed the ITD+ population of subreads to the next stage for codon mutation analysis. A list of the number of total subreads identified is listed in Supplementary Table 2. We identified ∼1000-10,000 subreads spanning the whole region between the ITD region and the furthest codon of interest (Y842) for codon analysis per sample.

For codon mutation analysis, we restricted our analysis to the 608, 691, 835, and 842 codons from reference sequence NM_004119 (Homo sapiens fms-related tyrosine kinase 3 (FLT3), mRNA) and then took the frequency of sequences obtained for each of these codons in the PCR amplicon of the healthy control and compared that to the frequency of sequences in each AML patient sample. A local quality filter that required exact matching of the codons before and after the codon of interest was used for filtering out low quality codon calls that might be due to sequencing error. We used the observed frequencies from the control sample for calculating the significance of the observed mutation in the AML patient samples. The p-value was calculated using a Poisson approximation considering the frequency observed in the control sample and the AML patient sample.^{35,36} Due to the potential statistical bias that could arise if the number of observed mutations was small in some cases, or if sequencing error frequencies differed between mutant and reference codon sequences, we only report the mutations using a conservative significance threshold of p< 1x10⁻⁷.

To further refine our search for mutations underlying relapse in these patients, we considered only those mutations that were in *cis* to an ITD, as defined on being on the same single DNA molecule sequence read. These mutations at both baseline and relapse are listed in Table 2. Finally, we considered only mutations with a frequency greater than the threshold of 2% as candidate contributors to relapse, as the frequency of mutations in the normal control was less than 2% at all codons examined.

### Molecular docking

The molecular docking was performed using Autodock 4.2 package³⁷. FLT-ITD structure (residue 587-947) was prepared from the protein data bank entry 1RJB¹⁹. All bound waters were removed from the protein. The structure was then added for hydrogens, and partial atomic charges were assigned using AutoDockTools³⁷. Residue K644, F830, F691 and E661 were selected as flexible residues. The coordinates of AC220 were generated using the Dundee PROGRD2 server³⁸, and its initial conformation was energy-minimized by the GROMACS force field. The Gasteiger charges were then assigned to the ligand using ADT. Seven torsion bonds were defined rotable during the docking procedure. The ligand was put into the kinase ATP binding pocket and manually aligned to avoid atom clashes. A three dimensional grid box (dimensions: 60×30×60, grid spacing: 0.375Å, centered at ligand) defining the search space was then created by AutoGrid4.2³⁷. Two hundred runs of Larmarckian Genetic Algorithm were performed to optimize the ligand-protein interactions. The solutions were clustered according to the root mean standard deviation values, and ranked by the binding free energy. Only the lowest-energy solution was analyzed. The inhibition constant of AC220 is estimated as 19.25nM (binding free energy: -10.51kcal/mol), which is in good agreement with the experimental data.

### References

1. Thiede, C., et al. Analysis of FLT3-activating mutations in 979 patients with acute myelogenous leukemia: association with FAB subtypes and identification of subgroups with poor prognosis. Blood 99, 4326-4335 (2002).
2. Kelly, L.M., et al. FLT3 internal tandem duplication mutations associated with human acute myeloid leukemias induce myeloproliferative disease in a murine bone marrow transplant model. Blood 99, 310-318 (2002).
3. Lee, B.H., et al. FLT3 internal tandem duplication mutations induce myeloproliferative or lymphoid disease in a transgenic mouse model. Oncogene 24, 7882-7892 (2005).
4. Shih, L.Y., et al. Acquisition of FLT3 or N-ras mutations is frequently associated with progression of myelodysplastic syndrome to acute myeloid leukemia. Leukemia 18, 466-475 (2004).
5. McCormick, S.R., et al. FLT3 mutations at diagnosis and relapse in acute myeloid leukemia: cytogenetic and pathologic correlations, including cuplike blast morphology. Arch Pathol Lab Med 134, 1143-1151 (2010).
6. Knapper, S., et al. A phase 2 trial of the FLT3 inhibitor lestaurtinib (CEP701) as first-line treatment for older patients with acute myeloid leukemia not considered fit for intensive chemotherapy. Blood 108, 3262-3270 (2006).
7. Fischer, T., et al. Phase IIB trial of oral Midostaurin (PKC412), the FMS-like tyrosine kinase 3 receptor (FLT3) and multi-targeted kinase inhibitor, in patients with acute myeloid leukemia and high-risk myelodysplastic syndrome with either wild-type or mutated FLT3. J Clin Oncol 28, 4339-4345 (2010).
8. De Angelo, D.J., et al. Phase II Evaluation of the Tyrosine Kinase Inhibitor MLN518 in Patients with Acute Myeloid Leukemia (AML) Bearing a FLT3 Internal Tandem Duplication (ITD) Mutation. ASH Annual Meeting Abstracts 104, 1792- (2004).
9. Heidel, F., et al. Clinical resistance to the kinase inhibitor PKC412 in acute myeloid leukemia by mutation of Asn-676 in the FLT3 tyrosine kinase domain. Blood 107, 293-300 (2006).
10. Metzelder, S., et al. Compassionate use of sorafenib in FLT3-ITD-positive acute myeloid leukemia: sustained regression before and after allogeneic stem cell transplantation. Blood 113, 6567-6571 (2009).
11. Scholl, S., et al. Secondary resistance to sorafenib in two patients with acute myeloid leukemia (AML) harboring FLT3-ITD mutations. Ann Hematol.
12. Zarrinkar, P.P., et al. AC220 is a uniquely potent and selective inhibitor of FLT3 for the treatment of acute myeloid leukemia (AML). Blood 114, 2984-2992 (2009).
13. Cortes, J., et al. A Phase II Open-Label, AC220 Monotherapy Efficacy (ACE) Study In Patients With Acute Myeloid Leukemia (AML) WITH FLT3-ITD Activating Mutations: Interim Results. in 16th Congress of the European Hematology Association (London, England, 2011).
14. Azam, M., Latek, R.R. & Daley, G.Q. Mechanisms of autoinhibition and STI-571/imatinib resistance revealed by mutagenesis of BCR-ABL. Cell 112, 831-843 (2003).
15. Shah, N.P., et al. Multiple BCR-ABL kinase domain mutations confer polyclonal resistance to the tyrosine kinase inhibitor imatinib (STI571) in chronic phase and blast crisis chronic myeloid leukemia. Cancer Cell 2, 117-125 (2002).
16. Travers, K.J., Chin, C.S., Rank, D.R., Eid, J.S. & Turner, S.W. A flexible and efficient template format for circular consensus sequencing and SNP detection. Nucleic Acids Res 38, e159.
17. Eid, J., et al. Real-time DNA sequencing from single polymerase molecules. Science 323, 133-138 (2009).
18. von Bubnoff, N., et al. FMS-like tyrosine kinase 3-internal tandem duplication tyrosine kinase inhibitors display a nonoverlapping profile of resistance mutations in vitro. Cancer Res 69, 3032-3041 (2009).
19. Griffith, J., et al. The structural basis for autoinhibition of FLT3 by the juxtamembrane domain. Mol Cell 13, 169-178 (2004).
20. Levinson, N.M., et al. A Src-like inactive conformation in the abl tyrosine kinase domain. PLoS Biol 4, e144 (2006).
21. Mol, C.D., et al. Structural basis for the autoinhibition and STI-571 inhibition of c-Kit tyrosine kinase. J Biol Chem 279, 31655-31663 (2004).
22. Hubbard, S.R., Wei, L., Ellis, L. & Hendrickson, W.A. Crystal structure of the tyrosine kinase domain of the human insulin receptor. Nature 372, 746-754 (1994).
23. Mol, C.D., et al. Structure of a c-kit product complex reveals the basis for kinase transactivation. J Biol Chem 278, 31461-31464 (2003).
24. Hubbard, S.R. Crystal structure of the activated insulin receptor tyrosine kinase in complex with peptide substrate and ATP analog. EMBO J 16, 5572-5581 (1997).
25. Pao, W., et al. Acquired resistance of lung adenocarcinomas to gefitinib or erlotinib is associated with a second mutation in the EGFR kinase domain. PLoS Med 2, e73 (2005).
26. Tamborini, E., et al. A new mutation in the KIT ATP pocket causes acquired resistance to imatinib in a gastrointestinal stromal tumor patient. Gastroenterology 127, 294-299 (2004).
27. Choi, Y.L., et al. EML4-ALK mutations in lung cancer that confer resistance to ALK inhibitors. N Engl J Med 363, 1734-1739 (2010).
28. Barbie, D.A. & Deangelo, D.J. Systemic mastocytosis: current classification and novel therapeutic options. Clin Adv Hematol Oncol 4, 768-775 (2006).
29. Notta, F., et al. Evolution of human BCR-ABL1 lymphoblastic leukaemia-initiating cells. Nature 469, 362-367 (2011).
30. Anderson, K., et al. Genetic variegation of clonal architecture and propagating cells in leukaemia. Nature 469, 356-361 (2011).
31. Kralovics, R., et al. Acquisition of the V617F mutation of JAK2 is a late genetic event in a subset of patients with myeloproliferative disorders. Blood 108, 1377-1380 (2006).
32. Nazarian, R., et al. Melanomas acquire resistance to B-RAF(V600E) inhibition by RTK or N-RAS upregulation. Nature 468, 973-977 (2010).
33. Johannessen, C.M., et al. COT drives resistance to RAF inhibition through MAP kinase pathway reactivation. Nature 468, 968-972 (2010).
34. Benson, G. Tandem repeats finder: a program to analyze DNA sequences. Nucleic Acids Res 27, 573-580 (1999).
35. Traut, H. A method for determining the statistical significance of mutation frequencies. Biometrical Journal 22, 73-78 (1980).
36. Zar, J.H. Statistical Significance of Mutation Frequencies, and the Power of Statistical Testing, Using the Poisson Distribution. Biometrical Journal 26, 83-88 (1984).
37. Morris, G.M., et al. AutoDock4 and AutoDockTools4: Automated docking with selective receptor flexibility. J Comput Chem 30, 2785-2791 (2009).
38. Schuttelkopf, A.W. & van Aalten, D.M. PRODRG: a tool for high-throughput crystallography of protein-ligand complexes. Acta Crystallogr D Biol Crystallogr 60, 1355-1363 (2004).

**Table 1**

| **Subject Number** | **Sex** | **Age (years)** | **Prior Therapy** | **Karyotype at Enrollment** | **Karyotype at Relapse** | **Percentage of Blasts in Relapse Sample** | **New Mutation at Relapse** | **ITD+ Clones with Mutation** | **Weeks on Study** |
|---|---|---|---|---|---|---|---|---|---|
| 1009-003 | F | 75 | 7+3 | 45∼54,XX,+3,+6,+7,+8,+13,+14,+21,+22[cp15]/46,XX[5] | 52,XX,+3,+6,+7,+8,+10,+12,+13[cp7]/46,XX[14] | 90% | D835F | 6/15 | 12 |
| 1009-007 | F | 64 | 7+3, HDAC | Normal | ND | 75% | D835Y | 5/10 | 10 |
| 1011-006 | M | 70 | 7+3, low dose cytarabine | Normal | ND | 10% | D835Y | 4/15 | 7 |
| 1011-007 | F | 56 | 7+3, HAM | Normal | 46,XX,del(11) (p?13p?15) [12]/46,XX[9] | 80% | F691L D835V | 4/24 5/24 | 8 |
| 1005-004 | F | 60 | cytarabine and mitoxantrone | Normal | Normal | 92% | F691L | 9/22 | 19 |
| 1005-006 | M | 43 | allogeneic stem cell transplant | 6,XY,t(1;15) (p22;q15) | ND | 59% | D835Y | 8/17 | 6 |
| 1005-007 | F | 59 | 7+3, HDAC | Normal | ND | 39% | D835V | 9/21 | 23 |
| 1005-009 | M | 68 | cytarabine and mitoxantrone | Normal | ND | 58% | D835Y | 8/14 | 18 |
| 1005-010 | M | 52 | 7+3, HDAC, mitoxantrone and etoposide | 46,XY,t(4;12) (q26;p11.2),t( 8;14) (q13;q11.2) | ND | 22% | F691L | 6/18 | 19 |

| | | | | | | | | | |
|---|---|---|---|---|---|---|---|---|---|
| 7+3 = low dose cytarabine x 7 days + 3 days anthracycline HAM = High dose cytarabine + mitoxantrone HDAC = High dose cytarabine ND = Not done | | | | | | | | | |

**Table 2**

| | | | Pre-Treatment | | | Retapse | | |
|---|---|---|---|---|---|---|---|---|
| Subject Number | Mutation | Native Codon | Alternative Codon | Observed alternative Codon Frequency | Total Number of ITD+ Sequences Sampled | Observed alternative Codon Frequency | Total Number of ITD- Sequences Sampled | Observed Alterantive Codon Frequency In Normal Control |
| 1009-003 | F691L | TTT | TTG | 4.7% | 1434 | 5.7% | 1306 | 1.2% |
| | D835Y | GAT | TAT | <2% | 1736 | 9.8% | 1382 | 1.2% |
| | D835V | GAT | GTT | <2% | 1736 | 3.6% | 1382 | 0.7% |
| | D835F | GAT | TTT | <2% | 1736 | 16.9% | 1382 | 0.1% |
| | | | | | | | | |
| 1009-007 | F691L | TTT | TTG | 6.4% | 1462 | 4.0% | 1079 | 1.2% |
| | D835Y | GAT | TAT | <2% | 2071 | 55.7% | 1229 | 1.2% |
| | | | | | | | | |
| 1011-006 | F691L | TTT | TTG | 5.3% | 603 | 4.7% | 493 | 1.2% |
| | D835Y | GAT | TAT | <2% | 700 | 38.4% | 380 | 1.2% |
| | | | | | | | | |
| 1011-007 | F691L | TTT | TTG | 5.8% | 1367 | 12.8% | 2748 | 1.2% |
| | F691L | TTT | CTT | <2% | 1367 | 3.7% | 2748 | 0.7% |
| | D835Y | GAT | TAT | <2% | 1478 | 5.2% | 3532 | 1.2% |
| | D835V | GAT | GTT | <2% | 1478 | 28.2% | 3532 | 0.7% |

| | | | | | | | | |
|---|---|---|---|---|---|---|---|---|
| All p-values <1x10⁻⁷ for alternative codon frequencies >2% | | | | | | | | |

**Supplementary Table 1**

| **Subject Number** | **New Mutation at Relapse** | **Pre-Treatment ITD-Clones with Mutation** |
|---|---|---|
| 1009-003 | D835F | 0/13 |
| 1009-007 | D835Y | 0/14 |
| 1011-006 | D835Y | 0/12 |
| 1011-007 | F691L | 0/11 |
| | D835V | 0/11 |
| 1005-004 | F691L | 0/22 |
| 1005-006 | D835Y | 0/15 |
| 1005-007 | D835V | 0/11 |
| 1005-009 | D835Y | 0/11 |
| 1005-010 | F691L | 0/24 |

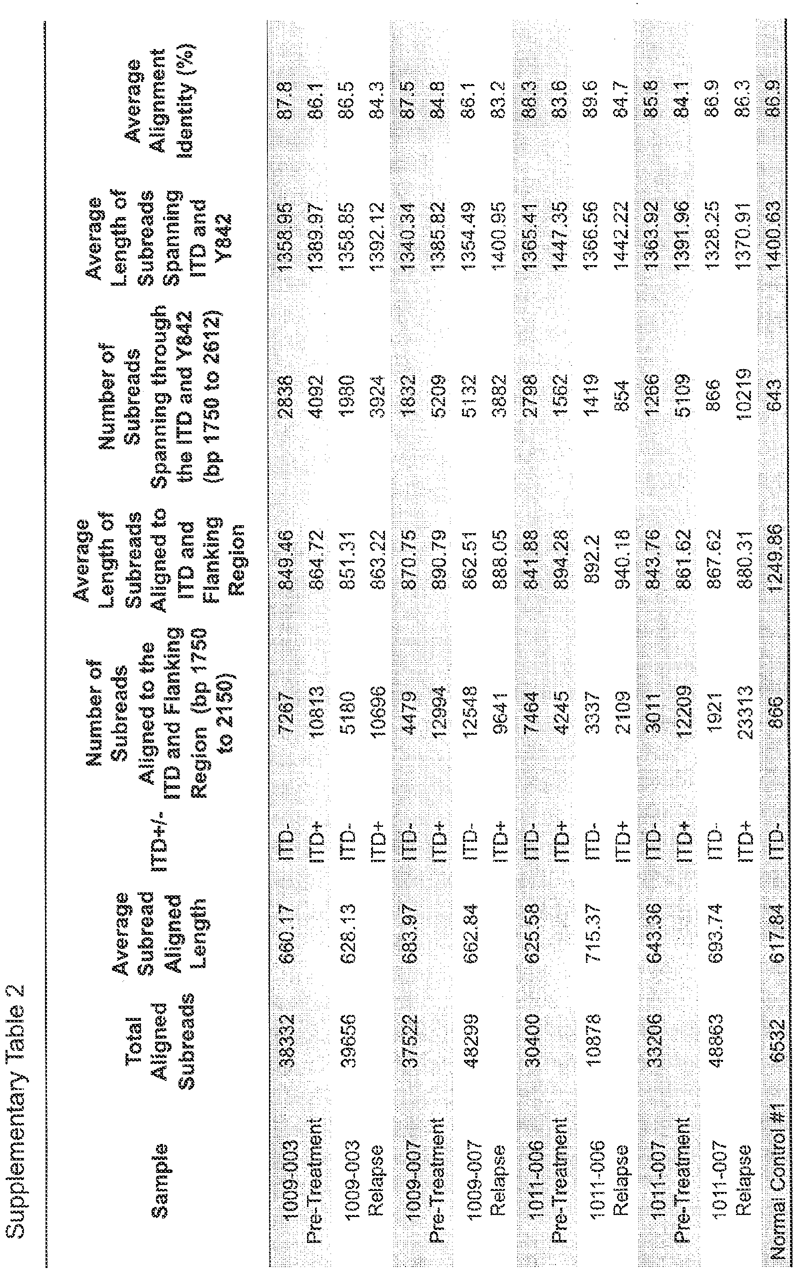

SEQ ID NO:1 Example of a FLT3 polynucleotide cDNA sequence (Accession number NM_004119.2) CDS:83..3064 (Start ATG indicated in bold)

1 acctgcagcg cgaggcgcgc cgctccaggc ggcatcgcag ggctgggccg gcgcggcctg
61 gggaccccgg gctccggagg ccatgccggc gttggcgcgc gacggcggcc agctgccgct
121 gctcgttgtt ttttctgcaa tgatatttgg gactattaca aatcaagatc tgcctgtgat
181 caagtgtgtt ttaatcaatc ataagaacaa tgattcatca gtggggaagt catcatcata
241 tcccatggta tcagaatccc cggaagacct cgggtgtgcg ttgagacccc agagctcagg
301 gacagtgtac gaagctgccg ctgtggaagt ggatgtatct gcttccatca cactgcaagt
361 gctggtcgac gccccaggga acatttcctg tctctgggtc tttaagcaca gctccctgaa
421 ttgccagcca cattttgatt tacaaaacag aggagttgtt tccatggtca ttttgaaaat
481 gacagaaacc caagctggag aatacctact ttttattcag agtgaagcta ccaattacac
541 aatattgttt acagtgagta taagaaatac cctgctttac acattaagaa gaccttactt
601 tagaaaaatg gaaaaccagg acgccctggt ctgcatatct gagagcgttc cagagccgat
661 cgtggaatgg gtgctttgcg attcacaggg ggaaagctgt aaagaagaaa gtccagctgt
721 tgttaaaaag gaggaaaaag tgcttcatga attatttggg acggacataa ggtgctgtgc
781 cagaaatgaa ctgggcaggg aatgcaccag gctgttcaca atagatctaa atcaaactcc
841 tcagaccaca ttgccacaat tatttcttaa agtaggggaa cccttatgga taaggtgcaa
901 agctgttcat gtgaaccatg gattcgggct cacctgggaa ttagaaaaca aagcactcga
961 ggagggcaac tactttgaga tgagtaccta ttcaacaaac agaactatga tacggattct
1021 gtttgctttt gtatcatcag tggcaagaaa cgacaccgga tactacactt gttcctcttc
1081 aaagcatccc agtcaatcag ctttggttac catcgtagaa aagggattta taaatgctac
1141 caattcaagt gaagattatg aaattgacca atatgaagag ttttgttttt ctgtcaggtt
1201 taaagcctac ccacaaatca gatgtacgtg gaccttctct cgaaaatcat ttccttgtga
1261 gcaaaagggt cttgataacg gatacagcat atccaagttt tgcaatcata agcaccagcc
1321 aggagaatat atattccatg cagaaaatga tgatgcccaa tttaccaaaa tgttcacgct
1381 gaatataaga aggaaacctc aagtgctcgc agaagcatcg gcaagtcagg cgtcctgttt
1441 ctcggatgga tacccattac catcttggac ctggaagaag tgttcagaca agtctcccaa
1501 ctgcacagaa gagatcacag aaggagtctg gaatagaaag gctaacagaa aagtgtttgg
1561 acagtgggtg tcgagcagta ctctaaacat gagtgaagcc ataaaagggt tcctggtcaa
1621 gtgctgtgca tacaattccc ttggcacatc ttgtgagacg atccttttaa actctccagg
1681 ccccttccct ttcatccaag acaacatctc attctatgca acaattggtg tttgtctcct
1741 cttcattgtc gttttaaccc tgctaatttg tcacaagtac aaaaagcaat ttaggtatga
1801 aagccagcta cagatggtac aggtgaccgg ctcctcagat aatgagtact tctacgttga

1861 tttcagagaa tatgaatatg atctcaaatg ggagtttcca agagaaaatt tagagtttgg
1921 gaaggtacta ggatcaggtg cttttggaaa agtgatgaac gcaacagctt atggaattag
1981 caaaacagga gtctcaatcc aggttgccgt caaaatgctg aaagaaaaag cagacagctc
2041 tgaaagagag gcactcatgt cagaactcaa gatgatgacc cagctgggaa gccacgagaa
2101 tattgtgaac ctgctggggg cgtgcacact gtcaggacca atttacttga tttttgaata
2161 ctgttgctat ggtgatcttc tcaactatct aagaagtaaa agagaaaaat ttcacaggac
2221 ttggacagag attttcaagg aacacaattt cagtttttac cccactttcc aatcacatcc
2281 aaattccagc atgcctggtt caagagaagt tcagatacac ccggactcgg atcaaatctc
2341 agggcttcat gggaattcat ttcactctga agatgaaatt gaatatgaaa accaaaaaag
2401 gctggaagaa gaggaggact tgaatgtgct tacatttgaa gatcttcttt gctttgcata
2461 tcaagttgcc aaaggaatgg aatttctgga atttaagtcg tgtgttcaca gagacctggc
2521 cgccaggaac gtgcttgtca cccacgggaa agtggtgaag atatgtgact ttggattggc
2581 tcgagatatc atgagtgatt ccaactatgt tgtcaggggc aatgcccgtc tgcctgtaaa
2641 atggatggcc cccgaaagcc tgtttgaagg catctacacc attaagagtg atgtctggtc
2701 atatggaata ttactgtggg aaatcttctc acttggtgtg aatccttacc ctggcattcc
2761 ggttgatgct aacttctaca aactgattca aaatggattt aaaatggatc agccatttta
2821 tgctacagaa gaaatataca ttataatgca atcctgctgg gcttttgact caaggaaacg
2881 gccatccttc cctaatttga cttcgttttt aggatgtcag ctggcagatg cagaagaagc
2941 gatgtatcag aatgtggatg gccgtgtttc ggaatgtcct cacacctacc aaaacaggcg
3001 acctttcagc agagagatgg atttggggct actctctccg caggctcagg tcgaagattc
3061 gtagaggaac aatttagttt taaggacttc atccctccac ctatccctaa caggctgtag
3121 attaccaaaa caagattaat ttcatcacta aaagaaaatc tattatcaac tgctgcttca
3181 ccagactttt ctctagaagc tgtctgcgtt tactcttgtt ttcaaaggga cttttgtaaa
3241 atcaaatcat cctgtcacaa ggcaggagga gctgataatg aactttattg gagcattgat
3301 ctgcatccaa ggccttctca ggctggcttg agtgaattgt gtacctgaag tacagtatat
3361 tcttgtaaat acataaaaca aaagcatttt gctaaggaga agctaatatg attttttaag
3421 tctatgtttt aaaataatat gtaaattttt cagctattta gtgatatatt ttatgggtgg
3481 gaataaaatt tctactacag aattgcccat tattgaatta tttacatggt ataattaggg
3541 caagtcttaa ctggagttca cgaaccccct gaaattgtgc acccatagcc acctacacat
3601 tccttccaga gcacgtgtgc ttttacccca agatacaagg aatgtgtagg cagctatggt
3661 tgtcacagcc taagatttct gcaacaacag gggttgtatt gggggaagtt tataatgaat
3721 aggtgttcta ccataaagag taatacatca cctagacact ttggcggcct tcccagactc
3781 agggccagtc agaagtaaca tggaggatta gtattttcaa taaagttact cttgtcccca

3841 caaaaaaa

SEQ ID NO:2 Example of a FLT3 polypeptide sequence; accession number

NP_004110.2

1 mpalardggq lpllvvfsam ifgtitnqdl pvikcvlinh knndssvgks ssypmvsesp

61 edlgcalrpq ssgtvyeaaa vevdvsasit lqvlvdapgn isclwvfkhs slncqphfdl

121 qnrgvvsmvi lkmtetqage yllfiqseat nytilftvsi rntllytlrr pyfrkmenqd

181 alvcisesvp epivewvlcd sqgesckees pavvkkeekv lhelfgtdir ccarnelgre

241 ctrlftidln qtpqttlpql flkvgeplwi rckavhvnhg fgltwelenk aleegnyfem

301 stystnrtmi rilfafvssv arndtgyytc ssskhpsqsa lvtivekgfi natnssedye

361 idqyeefcfs vrfkaypqir ctwtfsrksf pceqkgldng ysiskfcnhk hqpgeyifha

421 enddaqftkm ftlnirrkpq vlaeasasqa scfsdgyplp swtwkkcsdk spncteeite

481 gvwnrkanrk vfgqwvssst lnmseaikgf lvkccaynsl gtscetilln spgpfpfiqd

541 nisfyatigv cllfivvltl lichkykkqf ryesqlqmvq vtgssdneyf yvdfreyeyd

601 lkwefprenl efgkvlgsga fgkvmnatay gisktgvsiq vavkmlkeka dsserealms

661 elkmmtqlgs henivnllga ctlsgpiyli feyccygdll nylrskrekf hrtwteifke

721 hnfsfyptfq shpnssmpgs revqihpdsd qisglhgnsf hsedeieyen qkrleeeedl

781 nvltfedllc fayqvakgme flefkscvhr dlaarnvlvt hgkvvkicdf glardimsds

841 nyvvrgnarl pvkwmapesl fegiytiksd vwsygillwe ifslgvnpyp gipvdanfyk

901 liqngfkmdq pfyateeiyi imqscwafds rkrpsfpnlt sflgcqlada eeamyqnvdg

961 rvsecphtyq nrrpfsremd lgllspqaqv eds

## Claims

1. A method of identifying an AML patient undergoing treatment with AC220 that has an increased likelihood of relapse, wherein the patient has an initial activating mutation in a FLT3 gene, the method comprising detecting the presence of at least a second mutation in the FLT3 gene in an AML cell sample from the patient, wherein the second mutation results in an amino acid substitution at position F691, D835, or Y842 of FLT3.

2. The method of claim 1, wherein the substitution is at F691 or D835.

3. The method of claim 1, wherein the initial activating mutation is an in tandem duplication (ITD) mutation.

4. The method of claim 1, wherein the method comprises determining a mutation in a FLT3 gene at a codon that encodes F691, D835, or Y842.

5. The method of claim 4, wherein the method comprises sequencing a FLT3 nucleic acid amplified from the AML cell sample from the region of the FLT3 gene that comprises the codon.

6. The method of claim 1, wherein the mutation is at D835.

7. The method of claim 6, wherein the mutation is D835Y, D835V, or D835F.

8. The method of claim 6, wherein the mutation is D835F.

9. The method of claim 1, wherein the mutation is at F691.

10. The method of claim 9, wherein the mutation is F691L

11. The method of claim 1, wherein the AML cells comprise a mutation at position F691 and a mutation at position D835.

12. The method of claim 1, wherein the AML cell sample is from blood.

13. The method of claim 1, wherein the AML cell sample is from bone marrow.

## Patentansprüche

1. Verfahren zum Identifizieren eines AML-Patienten, der eine Behandlung mit AC220 durchläuft, der eine erhöhte Wahrscheinlichkeit eines Rezidivs hat, wobei der Patient eine anfängliche aktivierende Mutation in einem FLT3-Gen hat, wobei das Verfahren das Erfassen des Vorhandenseins von mindestens einer zweiten Mutation in dem FLT3-Gen in einer AML-Zellprobe aus dem Patienten umfasst, wobei die zweite Mutation zu einer Aminosäuresubstitution an Position F691, D835 oder Y842 von FLT3 führt.

2. Verfahren nach Anspruch 1, wobei die Substitution an F691 oder D835 ist.

3. Verfahren nach Anspruch 1, wobei die anfängliche aktivierende Mutation eine Tandemverdopplungs (ITD)-mutation ist.

4. Verfahren nach Anspruch 1, wobei das Verfahren das Bestimmen einer Mutation in einem FLT3-Gen an einem Codon umfasst, das F691, D835 oder Y842 codiert.

5. Verfahren nach Anspruch 4, wobei das Verfahren das Sequenzieren einer FLT3-Nukleinsäure umfasst, die aus der AML-Zellprobe von der Region des FLT3-Gens, das das Codon umfasst, amplifiziert ist.

6. Verfahren nach Anspruch 1, wobei die Mutation an D835 ist.

7. Verfahren nach Anspruch 6, wobei die Mutation D835Y, D835V oder D835F ist.

8. Verfahren nach Anspruch 6, wobei die Mutation D835F ist.

9. Verfahren nach Anspruch 1, wobei die Mutation an F691 ist.

10. Verfahren nach Anspruch 9, wobei die Mutation F691L ist.

11. Verfahren nach Anspruch 1, wobei die AML-Zellen eine Mutation an Position F691 und eine Mutation an Position D835 umfassen.

12. Verfahren nach Anspruch 1, wobei die AML-Zellprobe aus Blut ist.

13. Verfahren nach Anspruch 1, wobei die AML-Zellprobe aus Knochenmark ist.

## Revendications

1. Procédé d'identification d'un patient AML faisant l'objet d'un traitement avec AC220 qui présente un risque accru de rechute, dans lequel le patient a une mutation activatrice initiale dans un gène FLT3, le procédé comprenant la détection de la présence d'au moins une seconde mutation dans le gène FLT3 dans un échantillon cellulaire AML du patient, dans lequel la seconde mutation a pour résultat une substitution d'acide aminé à la position F691, D835, ou Y842 de FLT3.

2. Procédé selon la revendication 1, dans lequel la substitution est à F691 ou D835.

3. Procédé selon la revendication 1, dans lequel la mutation activatrice initiale est une mutation à duplication en tandem (ITD).

4. Procédé selon la revendication 1, dans lequel le procédé comprend la détermination d'une mutation dans un gène FLT3 à un codon qui encode F691, D835, ou Y842.

5. Procédé selon la revendication 4, dans lequel le procédé comprend le séquençage d'un acide nucléique FLT3, amplifié de l'échantillon cellulaire AML, de la région du gène FLT3 qui comprend le codon.

6. Procédé selon la revendication 1, dans lequel la mutation est à D835.

7. Procédé selon la revendication 6, dans lequel la mutation est D835Y, D835V, ou D835F.

8. Procédé selon la revendication 6, dans lequel la mutation est D835F.

9. Procédé selon la revendication 1, dans lequel la mutation est à F691.

10. Procédé selon la revendication 9, dans lequel la mutation est F691L.

11. Procédé selon la revendication 1, dans lequel les cellules AML comprennent une mutation à la position F691 et une mutation à la position D835.

12. Procédé selon la revendication 1, dans lequel l'échantillon cellulaire AML provient du sang.

13. Procédé selon la revendication 1, dans lequel l'échantillon cellulaire AML provient de la moelle osseuse.
